(19) **Europäisches Patentamt / European Patent Office / Office européen des brevets**

(11) **EP 4 621 065 A1**

(12) **EUROPEAN PATENT APPLICATION**

(43) Date of publication:
24.09.2025 Bulletin 2025/39

(51) International Patent Classification (IPC):
**C12Q 1/42** (2006.01)

(21) Application number: 25164075.1

(22) Date of filing: **17.03.2025**

(52) Cooperative Patent Classification (CPC):
**C12Q 1/42; G01N 33/573;** G01N 2333/916

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.03.2024 JP 2024044209**

(71) Applicant: **SYSMEX CORPORATION**
**Kobe-shi**
**Hyogo 651-0073 (JP)**

(72) Inventors:
• **ONISHI, Mami**
**Kobe-shi, Hyogo, 651-0073 (JP)**

• **KIRIYAMA, Maria**
**Kobe-shi, Hyogo, 651-0073 (JP)**
• **MUTO, Masaya**
**Kobe-shi, Hyogo, 651-0073 (JP)**
• **YAMASHITA, Kazuto**
**Kobe-shi, Hyogo, 651-0073 (JP)**
• **TAMADA, Eiya**
**Kobe-shi, Hyogo, 651-0073 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **METHOD FOR MEASURING ACTIVITY OR CONCENTRATION OF ALKALINE PHOSPHATASE CONTAINED IN EXTRACELLULAR VESICLE IN SAMPLE, POLYPEPTIDE MOLECULE, METHOD FOR PRODUCING THE SAME, AND CALIBRATOR**

(57) [Problem] To provide a calibrator used for measuring activity or concentration of alkaline phosphatase contained in an extracellular vesicle in a sample, and a measurement method using the calibrator.

[Solution] To solve the above problem, by acquiring an activity value or concentration of alkaline phosphatase contained in an extracellular vesicle in a sample using a calibrator containing a polypeptide molecule having a series of amino acid sequences including an amino acid sequence of alkaline phosphatase and an amino acid sequence of a membrane protein present on a surface of the extracellular vesicle.

FIG. 4A

EP 4 621 065 A1

**Description**

[Technical field]

The present invention relates to a method for measuring an activity or concentration of alkaline phosphatase (ALP) contained in an extracellular vesicle (EV) in a sample. The present invention relates to a polypeptide molecule used for measuring an activity or concentration of ALP contained in EV in a sample, and a method for producing the same. The present invention relates to a calibrator used for measuring an activity or concentration of ALP contained in EV in a sample.

[BACKGROUND]

EV is a nano-sized (several tens of nanometers to several hundreds of nanometers) membrane vesicle surrounded by a lipid double membrane, and is secreted from almost all cells. The EV is present in various biological samples such as blood and urine. The interior of the EVs contains various substances such as cell-derived proteins, DNA, mRNA, miRNA, lipids, sugar chains, and metabolites. In recent years, it has been reported that a substance contained in EV functions as an intercellular signaling molecule and participates in various physiological or pathological processes. For example, Patent Document 1 describes that EV participates in calcification of blood vessel.
Calcification in vivo mainly results from deposition of hydroxyapatite on collagen fibers. Calcification is essential for maintaining the physiological activity of bone and teeth, but when calcification occurs in blood vessels, it causes ischemic heart disease, cerebrovascular disorder, heart failure, and so forth. Therefore, it is important to establish an analysis method for discriminating calcification of blood vessels. For example, in Patent Document 1, in order to discriminate calcification of a blood vessel, EV is isolated from a sample, and a calcium salt contained in the EV is measured.

[Prior art reference]

[Patent Document]

[Patent document 1] U.S. Pat. No. 8,993,247

[Summary of the invention]

[Problem to be solved by the invention]

The present inventors have found that ALP present on a surface of EV participates in calcification of a blood vessel, and have established a method for measuring the activity of ALP contained in EV in a sample. On the other hand, when quantitatively measuring a test substance, a calibrator is usually used. A calibrator generally refers to a reagent containing a standard substance corresponding to a test substance. However, a suitable calibrator for measuring ALP contained in EV is not known. An object of the present invention is to provide a calibrator used for measuring an activity or concentration of ALP contained in EV, a measurement method using the calibrator, a standard substance contained in the calibrator, and a method for producing the same.

[Means for solving the problem]

The following inventions are provided.

[1] A method for measuring an activity of alkaline phosphatase contained in an extracellular vesicle in a sample, comprising the step of acquiring an activity value of alkaline phosphatase contained in the extracellular vesicle in the sample using a calibrator,
wherein the calibrator comprises a polypeptide molecule comprising an amino acid sequence of alkaline phosphatase and an amino acid sequence of a membrane protein present on a surface of the extracellular vesicle.
[2] The method according to [1], wherein the polypeptide molecule comprises an enzyme activity of alkaline phosphatase based on the polypeptide chain of the amino acid sequence of the alkaline phosphatase.
[3] The method according to [2], further comprising the step of measuring the activity of alkaline phosphatase contained in the extracellular vesicle in the sample and the activity of the polypeptide molecule in the calibrator,
wherein the activity value of alkaline phosphatase contained in the extracellular vesicle in the sample is acquired based on results of the measuring.
[4] The method according to [3] further comprising the step of:

forming on a solid phase a first complex comprising the extracellular vesicle in the sample and a capture antibody that specifically binds to the membrane protein contained in the extracellular vesicle;

measuring a first signal generated by contacting the extracellular vesicle in the first complex with a substrate for alkaline phosphatase;

forming on a solid phase a second complex comprising the polypeptide molecule in the calibrator and the capture antibody;

measuring a second signal generated by contacting the polypeptide molecule in the second complex with the substrate; and

acquiring an activity value of the alkaline phosphatase contained in the extracellular vesicle in the sample from a measured value of the first signal and a measured value of the second signal.

[5] The method according to [1] or [2], wherein

the alkaline phosphatase contained in the extracellular vesicle in the sample and the polypeptide molecule in the calibrator are each detected, and

a concentration of the alkaline phosphatase contained in the extracellular vesicle in the sample is acquired based on results of the detections.

[6] The method according to [5], wherein the polypeptide molecule is detected via a polypeptide chain represented by the amino acid sequence of the alkaline phosphatase.

[7] The method according to [5] or [6], further comprising:

forming on a solid phase a first complex comprising the extracellular vesicle in the sample, a capture antibody that specifically binds to a membrane protein contained in the extracellular vesicle, a detection antibody that specifically binds to alkaline phosphatase contained in the extracellular vesicle, and a labeling substance;

measuring a first signal generated by the labeling substance contained in the first complex;

forming on a solid phase a second complex comprising the polypeptide molecule in the calibrator, the capture antibody, the detection antibody, and the labeling substance;

measuring a second signal generated by the labeling substance contained in the second complex; and

acquiring an activity value of alkaline phosphatase contained in the extracellular vesicle in the sample from measured values of the first signal and the second signal.

[8] The method according to any of [1] to [7], wherein the calibrator comprises a homodimer consisting of two of the polypeptide molecules.

[9] The method according to any of [1] to [8], wherein the amino acid sequence of the membrane protein of the polypeptide molecule is an amino acid sequence selected from the group consisting of an extracellular domain of CD9, a region from an N-terminus to a position 20 of Annexin A1, an extracellular domain of CD63, an extracellular domain of CD81, an extracellular domain of Annexin A6, and an extracellular domain of Pit 1.

[10] The method according to any of [1] to [9], wherein the amino acid sequence of the alkaline phosphatase contained in the polypeptide molecule is an amino acid sequence selected from the group consisting of tissue non-specific alkaline phosphatase, small intestine alkaline phosphatase, placenta alkaline phosphatase, germ cell alkaline phosphatase, and fragments thereof.

[11] The method according to any of [1] to [10], wherein the polypeptide molecule comprises an amino acid sequence of a peptide linker between the amino acid sequence of the alkaline phosphatase and the amino acid sequence of the membrane protein.

[12] A polypeptide molecule for measuring activity or concentration of alkaline phosphatase contained in an extracellular vesicle in a sample,

the polypeptide molecule comprising a series of an amino acid sequence comprising an amino acid sequence of the alkaline phosphatase and an amino acid sequence of a membrane protein present on a surface of the extracellular vesicle.

[13] The polypeptide molecule according to [12], wherein the polypeptide chain represented by the amino acid sequence of alkaline phosphatase has enzyme activity of alkaline phosphatase.

[14] The polypeptide molecule according to [12] or [13], comprising an amino acid sequence of a peptide linker between the amino acid sequence of the alkaline phosphatase and the amino acid sequence of the membrane protein.

[15] The polypeptide molecule according to any of [12] to [14], wherein the amino acid sequence of the membrane protein of the polypeptide molecule is an amino acid sequence selected from the group consisting of an extracellular domain of CD9, a region from an N-terminus to a position 20 of Annexin A1, an extracellular domain of CD63, an extracellular domain of CD81, an extracellular domain of Annexin A6, and an extracellular domain of Pit 1.

[16] The polypeptide molecule according to any of [12] to [15], wherein the amino acid sequence of the alkaline phosphatase contained in the polypeptide molecule is an amino acid sequence selected from the group consisting of tissue non-specific alkaline phosphatase, small intestine alkaline phosphatase, placenta alkaline phosphatase, germ cell alkaline phosphatase, and fragments thereof.

[17] A homodimer comprising the polypeptide molecule according to any one of [12] to [16].

[18] A calibrator for measuring activity or concentration of alkaline phosphatase contained in an extracellular vesicle in a sample,

the calibrator comprising a polypeptide molecule comprising a series of amino acid sequences comprising an amino acid sequence of alkaline phosphatase and an amino acid sequence of a membrane protein present on a surface of an extracellular vesicle.

[19] The calibrator according to [18], wherein the polypeptide molecule has enzyme activity of alkaline phosphatase based on a polypeptide chain represented by the amino acid sequence of the alkaline phosphatase.

[20] The calibrator according to [18] or [19], wherein the polypeptide molecule comprises an amino acid sequence of a peptide linker between the amino acid sequence of the alkaline phosphatase and the amino acid sequence of the membrane protein.

[21] The calibrator according to any of [18] to [20], wherein the amino acid sequence of the membrane protein of the polypeptide molecule is an amino acid sequence selected from the group consisting of an extracellular domain of CD9, a region from an N-terminus to a position 20 of Annexin A1, an extracellular domain of CD63, an extracellular domain of CD81, an extracellular domain of Annexin A6, and an extracellular domain of Pit 1.

[22] The calibrator according to any of [18] to [21], wherein the amino acid sequence of alkaline phosphatase contained in the polypeptide molecule is an amino acid sequence selected from the group consisting of tissue non-specific alkaline phosphatase, small intestine alkaline phosphatase, placenta alkaline phosphatase, germ cell alkaline phosphatase, and fragments thereof.

[23] The calibrator according to any of [18] to [22], comprising a homodimer comprising two of the polypeptide molecules.

[24] A method for producing a polypeptide molecule for measuring activity or concentration of alkaline phosphatase contained in an extracellular vesicle in a sample, comprising:

culturing a cell into which an expression vector comprising a gene encoding a polypeptide molecule comprising a series of amino acid sequences comprising an amino acid sequence of alkaline phosphatase and an amino acid sequence of a membrane protein present on a surface of the extracellular vesicle is introduced; and acquiring the polypeptide molecule expressed by the cell.

[Effect of the Invention]

According to the present invention, there are provided a method for measuring an activity or concentration of ALP contained in EV using a calibrator, a calibrator used in the method, a polypeptide molecule contained in the calibrator, and a method for producing the polypeptide molecule.

[Brief Description of the Drawings]

[Fig. 1A] is a schematic diagram of a polypeptide molecule of the present embodiment.
[Fig. 1B] is a schematic diagram of a polypeptide molecule of the present embodiment including a peptide linker.
[Fig. 2] is a schematic diagram of a kit including a calibrator.
[Fig. 3A] is a schematic diagram of a kit including a set of calibrators.
[Fig. 3B] is a schematic diagram of a kit including a set of calibrators.
[Fig. 4A] is a schematic diagram of activity measurement of ALP contained in EV in a sample.
[Fig. 4B] is a schematic diagram of activity measurement of a polypeptide molecule in the calibrator of the present embodiment.
[Fig. 5A] is a schematic diagram of detection of ALP contained in EV in a sample.
[Fig. 5B] is a schematic diagram of detection of a polypeptide molecule in the calibrator of the present embodiment.
[Fig. 6] is a schematic diagram of an expression vector.
[Fig. 7] is a block diagram showing a configuration of an immunoassay device 1.
[Fig. 8A] is a flowchart showing an example of preparation of a calibration curve using the immunoassay device 1.
[Fig. 8B] is a flowchart showing an example of measurement of a sample using the immunoassay device 1.

[Fig. 9A] is a schematic diagram of expression vectors 1 to 8 used in a production example of a polypeptide molecule.

[Fig. 9B] is a schematic diagram of a polypeptide molecule expressed by a cell including the expression vectors 1 to 8.

[Fig. 10] is a chromatogram showing results of analysis of a culture supernatant containing the polypeptide molecule 1 by size exclusion column chromatography (SEC) and a graph showing results of measurement of ALP activity of each fraction obtained by SEC.

[Fig. 11] is a diagram showing results of analysis of a fraction containing the polypeptide molecule 1 by SDS-PAGE and CBB staining.

[Fig. 12] is a graph showing results of measuring ALP activity of polypeptide molecule 1 by immunoassay.

[Fig. 13A] is calibration curve 1 prepared based on the measured results of a calibrator containing polypeptide molecule 1.

[Fig. 13B] is a calibration curve 2 prepared based on the measured results of a calibrator containing the polypeptide molecule 1.

[Fig. 14A] is a graph showing results of measuring protein concentration of EV ($\mu$g/mL) in a sample derived from a patient having a history of cardiovascular disease, using a calibrator containing the polypeptide molecule 1.

[Fig. 14B] is a graph showing results of measuring the activity value (U/L) of ALP contained in EV in a sample derived from a patient having a history of cardiovascular disease using a calibrator containing the polypeptide molecule 1.

[Fig. 15] is a diagram showing results of analysis of each eluent containing the polypeptide molecules 2 to 5 by SDS-PAGE and CBB staining.

[Fig. 16A] is a graph showing results of measuring ALP activity of polypeptide molecule 2 by immunoassay.

[Fig. 16B] is a graph showing results of measuring ALP activity of polypeptide molecule 3 by immunoassay.

[Fig. 16C] is a graph showing results of measuring ALP activity of polypeptide molecule 4 by immunoassay.

[Fig. 16D] is a graph showing results of measuring ALP activity of polypeptide molecule 5 by immunoassay.

[Fig. 17A] is a calibration curve 3 prepared based on the measured results of a calibrator containing the polypeptide molecule 2.

[Fig. 17B] is a calibration curve 4 prepared based on the measured results of a calibrator containing the polypeptide molecule 2.

[Fig. 18A] is a calibration curve 5 prepared based on the measured results of a calibrator containing the polypeptide molecule 3.

[Fig. 18B] is a calibration curve 6 prepared based on the measured results of a calibrator containing the polypeptide molecule 3.

[Fig. 19A] is a calibration curve 7 prepared based on the measured results of a calibrator containing the polypeptide molecule 4.

[Fig. 19B] is a calibration curve 8 prepared based on the measured results of a calibrator containing the polypeptide molecule 4.

[Fig. 20A] is a calibration curve 9 prepared based on the measured results of a calibrator containing the polypeptide molecule 5.

[Fig. 20B] is a calibration curve 10 prepared based on the measured results of a calibrator containing the polypeptide molecule 5.

[Fig. 21] is a graph showing results of measuring ALP activity of polypeptide molecule 6 by immunoassay.

[Fig. 22] is a calibration curve 11 prepared based on the measured results of a calibrator containing the polypeptide molecule 6.

[Fig. 23] is a chromatogram showing results of analysis of a culture supernatant containing the polypeptide molecule 7 with SEC, and a graph showing results of measurement of ALP activity of each fraction obtained with SEC.

[Fig. 24] is a diagram showing results of analysis of fractions containing polypeptide molecules 7 by SDS-PAGE and CBB staining.

[Fig. 25A] is a calibration curve 12 prepared based on the measured results of a calibrator containing the polypeptide molecule 7.

[Fig. 25B] is a calibration curve 13 prepared based on the measured results of a calibrator containing the polypeptide molecule 7.

[Fig. 26] is a calibration curve 14 prepared based on the measured results of a calibrator containing the polypeptide molecule 8.

## DETAILED DESCRIPTION OF THE PREFERRED EMBODIMENTS

Normally, the concentration of a test substance in a sample is unknown, but by using a calibrator, it becomes possible to quantitatively measure the test substance. In the method for measuring the activity or concentration of ALP contained in EV in a sample of the present embodiment (hereinafter, also referred to as "measurement method of the present embodiment"), the activity value or concentration of ALP contained in EV in a sample is acquired using a calibrator.

The measurement target is EV containing ALP, but membrane proteins are also present on the surface of EV in addition to ALP. Therefore, the test substance in the measurement method of the present embodiment is more specifically EV having ALP and a membrane protein.

Generally, the standard substance contained in the calibrator is a substance having essentially the same or similar structure and properties as the test substance. The present inventors have found that a polypeptide molecule having a series of amino acid sequences including an amino acid sequence of ALP and an amino acid sequence of a membrane protein present on a surface of EV (hereinafter, also referred to as a "polypeptide molecule of the present embodiment") can be used as a standard substance corresponding to EV having ALP and a membrane protein, and have completed a calibrator. Hereinafter, the polypeptide molecule and the calibrator of the present embodiment will be described, and then the measurement method of the present embodiment will be described.

The polypeptide molecule of the present embodiment is a substance in which a plurality of amino acids are bound by peptide bonds according to an amino acid sequence thereof. That is, the polypeptide molecule of the present embodiment can be said to be a fusion protein composed of a series of polypeptide chains including an ALP portion and a membrane protein portion. "ALP portion" is a polypeptide chain represented by an amino acid sequence of ALP in the polypeptide molecule of the present embodiment. ALP that functions as an enzyme usually forms a homodimer and exists. The ALP portion in the polypeptide molecule of the present embodiment corresponds to the full length of ALP of a monomer or a fragment thereof. [Membrane protein portion" is a polypeptide chain represented by an amino acid sequence of a membrane protein in the polypeptide molecule of the present embodiment. In the polypeptide molecule of the present embodiment, the number of ALP moieties may be one or more. Preferably, in the polypeptide molecule of the present embodiment, the number of ALP portions is 1. In the polypeptide molecule of the present embodiment, the number of membrane protein portions may be one or more. Preferably, in the polypeptide molecule of the present embodiment, the number of membrane protein portions is 1 or 2. The polypeptide molecule of the present embodiment is prepared by genetic recombination technology as described later.

The amino acid sequence of ALP may be a full-length sequence or a partial sequence. The full-length sequence of ALP can be acquired from known databases such as GeneBank, PDB, EMBL, DDBJ. A partial sequence of ALP refers to an amino acid sequence of a polypeptide chain (i.e., an ALP fragment) composed of a plurality of consecutive amino acid residues in a full-length sequence of ALP. The partial sequence of ALP may be, for example, an amino acid sequence of an ALP fragment in which enzyme activity of ALP (hereinafter, also referred to as "ALP activity"), an amino acid sequence of an ALP fragment including an epitope recognized by a detection antibody described later, or the like. Examples of the amino acid sequence of an ALP fragment in which ALP activity is maintained include an amino acid sequence in which a secretion signal sequence and/or a glycosylphosphatidylinositol (GPI) addition signal sequence is removed from a full-length sequence of ALP. The amino acid sequence of ALP may be a sequence modified from an amino acid sequence of wild-type ALP as long as ALP activity is maintained or can bind to a detection antibody described later. The modification of an amino acid sequence refers to deletion, substitution, addition, or combination of one or more amino acid residues.

The polypeptide molecule of the present embodiment preferably has ALP activity depending on a polypeptide chain represented by an amino acid sequence of ALP. As described above, ALP that functions as an enzyme usually forms a homodimer and exists. Therefore, when the polypeptide molecule of the present embodiment has ALP activity, it is considered that ALP activity is exerted by forming a homodimer of two molecules of the polypeptide molecule of the present embodiment via an ALP moiety. The polypeptide molecule of the present embodiment is preferably detected by a detection antibody described later via a polypeptide chain represented by an amino acid sequence of ALP.

The type of ALP used for the ALP portion may be the same as or different from the ALP contained in EV in the sample. Examples of the type of ALP include tissue non-specific ALP, small intestine ALP, placenta ALP, and germ cell ALP. Among them, small intestine ALP (IAP) and tissue non-specific ALP (TNAP) are preferable. The origin of ALP is not particularly limited, and examples thereof include ALP derived from human, bovine, mouse, rat, dog, cat, rabbit, and the like. The amino acid sequence of ALP is particularly preferably the amino acid sequence of bovine small intestine-derived ALP (BIAP) and human TNAP. Examples of the BIAPs include BIAP I, BIAP II, BIAP III, BIAP IV, BIAP V, BIAP VI, BIAP VII and the like (see Manes T. et al. (1998) J.Biol.Chem., vol.273, pp.23353 - 23360, U.S. Pat. No. 6,406,899, and the like). When there are a plurality of ALP portions in the polypeptide molecule of the present embodiment, the types of ALP used for the ALP portions may be the same. Alternatively, the at least one ALP portion may be of a type different from the remaining ALP portions. The partial sequence of BIAP II and the full-length sequence of human TNAP are shown below.

[Amino acid sequence of BIAP II]

LIPAEEENPAFWNRQAAQALDVAKKLQPIQTAAKNVILFLGDGMGVPTVTATRI
LKGQMNGKLGPETPLAMDQFPYVALSKTYNVDRQVPDSAGTATAYLCGVKG
NYRTIGVSAAARYNQCNTTRGNEVTSVINRAKKAGKAVGVVTTTRVQHASPA
GAYAHTVNRNWYSDADLPADAQKNGCQDIAAQLVYNMDIDVILGGGRMYMF
PEGTPDPEYPDDASVNGVRKDKQNLVQEWQAKHQGAQYVWNRTALLQAAD
DSSVTHLMGLFEPADMKYNVQQDHTKDPTLAEMTEAALQVLSRNPRGFYLFV
EGGRIDHGHHDGKAYMALTEAIMFDNAIAKANELTSELDTLILVTADHSHVFSF
GGYTLRGTSIFGLAPGKALDSKSYTSILYGNGPGYALGGGSRPDVNGSTSEEPS
YRQQAAVPLASETHGGEDVAVFARGPQAHLVHGVQEETFVAHIMAFAGCVEP
YTDCNLPAPA (SEQ ID NO: 1)

[Amino acid sequence of human TNAP]

MISPFLVLAIGTCLTNSLVPEKEKDPKYWRDQAQETLKYALELQKLNTNVAKN
VIMFLGDGMGVSTVTAARILKGQLHHNPGEETRLEMDKFPFVALSKTYNTNAQ
VPDSAGTATAYLCGVKANEGTVGVSAATERSRCNTTQGNEVTSILRWAKDAG
KSVGIVTTTRVNHATPSAAYAHSADRDWYSDNEMPPEALSQGCKDIAYQLMH
NIRDIDVIMGGGRKYMYPKNKTDVEYESDEKARGTRLDGLDLVDTWKSFKPR
YKHSHFIWNRTELLTLDPHNVDYLLGLFEPGDMQYELNRNNVTDPSLSEMVVV
AIQILRKNPKGFFLLVEGGRIDHGHHEGKAKQALHEAVEMDRAIGQAGSLTSSE
DTLTVVTADHSHVFTFGGYTPRGNSIFGLAPMLSDTDKKPFTAILYGNGPGYKV
VGGERENVSMVDYAHNNYQAQSAVPLRHETHGGEDVAVFSKGPMAHLLHGV
HEQNYVPHVMAYAACIGANLGHCAPASSAGSLAAGPLLLALALYPLSVLF
(SEQ ID NO: 2)

The amino acid sequence of a membrane protein may be a full-length sequence or a partial sequence. The full-length sequence of a membrane protein can be acquired, for example, from the aforementioned known database. The partial sequence of a membrane protein refers to an amino acid sequence of a polypeptide chain (that is, a membrane protein fragment) composed of a plurality of consecutive amino acid residues in a full-length sequence of a membrane protein. The partial sequence of the membrane protein may be, for example, an amino acid sequence of an extracellular domain of the membrane protein, an amino acid sequence of a membrane protein fragment including an epitope recognized by a capture antibody described later, or the like. The amino acid sequence of the membrane protein may be a sequence modified from an amino acid sequence of a wild-type membrane protein as long as it can bind to the capture antibody described later. Amino acid sequence modifications are as described above.

The type of membrane protein used for the membrane protein portion can be selected from membrane proteins known to be present on the surface of EV. Examples of the membrane protein include CD9, CD63, CD81, CD82, CD53, CD37, Annexin A1 (Annexin I), Annexin A2, Annexin A4, Annexin A5 (Annexin V), Annexin A6 (Annexin VI), Annexin A7, Annexin A11, Pit 1, Pit 2, sortilin, Rab, Alix, Tsg101, selectin, lactadherin, integrin, flotillin, glycosylphosphatidylinositol, heatshock protein (HSP-60,HSP-70,HSP-A5,CCT2,HSP90, etc.), Matrix metalloproteinase (MMP-2, MMP-3, MMP-9, MMP-13, etc.), S100 protein family (S 100-A9, etc.), Major histocompatibility complex (MHC)-I, MHC-II, etc. Among them, CD9, CD63, CD81, Annexin A1, Annexin A2, Annexin A4, Annexin A5, Annexin A6, Annexin A7, Annexin A11, Pit 1, Pit 2, sortilin, and S100-A9 are preferable.

The amino acid sequence of a membrane protein is particularly preferably an amino acid sequence of an extracellular domain of CD9, a region from the N-terminus to the position 20 of Annexin A1, an extracellular domain of CD63, an extracellular domain of CD81, an extracellular domain of Annexin A6, and an extracellular domain of Pit 1. When there are a plurality of membrane protein portions in the polypeptide molecule of the present embodiment, the types of membrane proteins used for the membrane protein portions may be the same. Alternatively, the at least one membrane protein portion may be of a type different from the remaining membrane protein portions. The amino acid sequences of the extracellular domain of CD9, the full length of CD9, the region from the N-terminus to the position 20 of Annexin A1, the extracellular domain of CD63, and the extracellular domain of CD81 are shown below.

[Amino acid sequence of extracellular domain of CD9]

SHKDEVIKEVQEFYKDTYNKLKTKDEPQRETLKAIHYALNCCGLAGGVEQFISD
ICPKKDVLETFTVKSCPDAIKEVFDNKFHI (SEQ ID NO: 3)

[Full-length amino acid sequence of CD9]

MPVKGGTKCIKYLLFGFNFIFWLAGIAVLAIGLWLRFDSQTKSIFEQETNNNNSS
FYTGVYILIGAGALMMLVGFLGCCGAVQESQCMLGLFFGFLLVIFAIEIAAAIW
GYSHKDEVIKEVQEFYKDTYNKLKTKDEPQRETLKAIHYALNCCGLAGGVEQF
ISDICPKKDVLETFTVKSCPDAIKEVFDNKFHIIGAVGIGIAVVMIFGMIFSMILCC
AIRRNREMV (SEQ ID NO: 4)

[Amino acid sequence of extracellular domain of CD63]

AGYVFRDKVMSEFNNNFRQQMENYPKNNHTASILDRMQADFKCCGAANYTD
WEKIPSMSKNRVPDSCCINVTVGCGINFNEKAIHKEGCVEKIGGWLRKNV (SEQ
ID NO: 5)

[Amino acid sequence of extracellular domain of CD81]

FVNKDQIAKDVKQFYDQALQQAVVDDDANNAKAVVKTFHETLDCCGSSTLTA
LTTSVLKNNLCPSGSNIISNLFKEDCHQKIDDLFSGK (SEQ ID NO: 6)

[Amino acid sequence of region from N-terminus to position 20 of Annexin A1]
MAMVSEFLKQAWFIENEEQE (SEQ ID NO: 7)

In the amino acid sequence of the polypeptide molecule of the present embodiment, an amino acid residue at the C-terminus of an amino acid sequence of a membrane protein may be followed by an amino acid residue at the N-terminus of an amino acid sequence of ALP. That is, in the polypeptide molecule of the present embodiment, the amino acid residue at the C-terminus of the membrane protein portion and the amino acid residue at the N-terminus of the ALP portion may be bound by peptide bonds. Furthermore/or alternatively, in the amino acid sequence of the polypeptide molecule of the present embodiment, the amino acid residue at the C-terminus of the amino acid sequence of ALP may be followed by the amino acid residue at the N-terminus of the amino acid sequence of a membrane protein. That is, in the polypeptide molecule of the present embodiment, the amino acid residue at the C-terminus of the ALP portion and the amino acid residue at the N-terminus of the membrane protein portion may be bound by peptide binding.

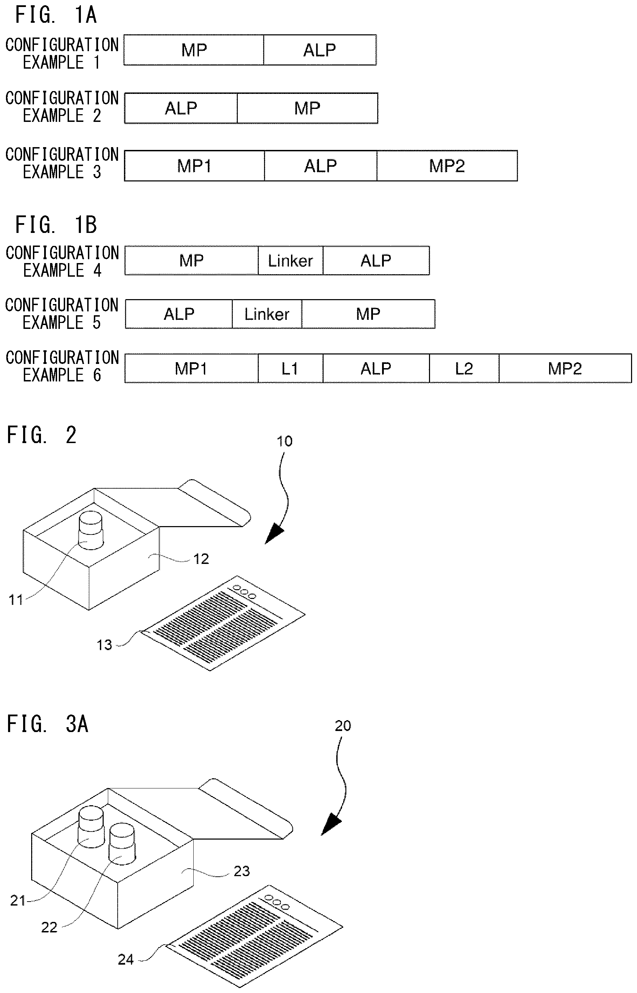

An example of the polypeptide molecule of the present embodiment is shown in Fig. 1A, but the present invention is not limited to these examples. In the figure, each of configuration examples 1 to 3 represents a polypeptide molecule of the present embodiment. "MP" represents a membrane protein portion, "MP1" represents a first membrane protein portion, "MP2" represents a second membrane protein portion, and "ALP" represents an ALP portion. The first membrane protein portion and the second membrane protein portion may be the same or different.

In the polypeptide molecule of Configuration Example 1, an amino acid residue at the C-terminus of a membrane protein portion and an amino acid residue at the N-terminus of an ALP portion are bound by a peptide bond. In the polypeptide molecule of Configuration Example 2, an amino acid residue at the C-terminus of the ALP portion and an amino acid residue at the N-terminus of the membrane protein portion are bound by a peptide bond. In the polypeptide molecule of Configuration Example 3, an amino acid residue at the C-terminus of the first membrane protein portion and an amino acid residue at the N-terminus of the ALP portion are bound by a peptide bond. Further, an amino acid residue at the C-terminus of the ALP portion and an amino acid residue at the N-terminus of the second membrane protein portion are bound by peptide bonds.

The amino acid sequence of the polypeptide molecule of the present embodiment may include an amino acid sequence of a peptide linker between an amino acid sequence of a membrane protein and an amino acid sequence of ALP. For example, in the amino acid sequence of the polypeptide molecule of the present embodiment, the amino acid residue at the N-terminus of the amino acid sequence of the peptide linker may be subsequent to the amino acid residue at the C-terminus of the amino acid sequence of the membrane protein, and the amino acid residue at the N-terminus of the amino acid sequence of ALP may be subsequent to the amino acid residue at the C-terminus of the amino acid sequence of the peptide linker. That is, in the polypeptide molecule of the present embodiment, the amino acid residue at the C-terminus of the membrane protein portion and the amino acid residue at the N-terminus of the peptide linker may be bound by peptide bonds, and the amino acid residue at the C-terminus of the peptide linker and the amino acid residue at the N-terminus of the ALP portion may be bound by peptide bonds. Furthermore/or alternatively, in the amino acid sequence of the polypeptide molecule of the present embodiment, the amino acid residue at the N-terminus of the amino acid sequence of the peptide linker may be subsequent to the amino acid residue at the C-terminus of the amino acid sequence of the ALP, and the amino acid residue at the N-terminus of the amino acid sequence of the membrane protein may be subsequent to the amino acid residue at the C-terminus of the amino acid sequence of the peptide linker. That is, in the polypeptide molecule of the present embodiment, the amino acid residue at the C-terminus of the ALP portion and the amino acid residue at the N-terminus of the peptide linker may be bound by peptide binding, and the amino acid residue at the C-terminus of the peptide linker and the amino acid residue at the N-terminus of the membrane protein portion may be bound by peptide binding.

The amino acid sequence of the peptide linker is not particularly limited as long as it does not affect the binding ability to the capture antibody and the detection antibody, and the ALP activity. The length of the peptide linker is not particularly limited, and is, for example, 3 to 20 amino acid residues. A preferred peptide linker is any one selected from a GS1 linker, a GS2 linker, a GS3 linker, an EK1 linker, an EK2 linker, and an EK3 linker represented by the following amino acid sequence. When there are a plurality of peptide linkers in the polypeptide molecule of the present embodiment, the types of the peptide linkers may be the same. Alternatively, the at least one peptide linker may be of a type different from the remaining peptide linker.

GS1 linker: GGGGS (SEQ ID NO: 8)
GS2 linker: GGGGSGGGGS (SEQ ID NO: 9)
GS3 linker: GGGGSGGGGSGGGGS (SEQ ID NO: 10)
EK1 linker: EAAAK (SEQ ID NO: 11)
EK2 linker: EAAAKEAAAK (SEQ ID NO: 12)
EK3 linker: EAAAKEAAAKEAAAK (SEQ ID NO: 13)

An example of the polypeptide molecule of the present embodiment including a peptide linker is shown in Fig. 1B, but the present invention is not limited to these examples. In the figure, each of Structure Examples 4 to 6 represents a polypeptide molecule of the present embodiment. "MP", "MP1", "MP2", and "ALP" are the same as those in Fig. 1A. "Linker" represents a peptide linker, "L1" represents a first peptide linker, and "L2" represents a second peptide linker. The first peptide linker and the second peptide linker may be the same or different.

In the polypeptide molecule of Configuration Example 4, an amino acid residue at the C-terminus of a membrane protein portion and an amino acid residue at the N-terminus of a peptide linker are bound by peptide bonds. Further, an amino acid residue at the C-terminus of the peptide linker and an amino acid residue at the N-terminus of the ALP portion are bound by a peptide bond. In the polypeptide molecule of Configuration Example 5, an amino acid residue at the C-terminus of the ALP portion and an amino acid residue at the N-terminus of the peptide linker are bound by a peptide bond. Further, an amino acid residue at the C-terminus of the peptide linker and an amino acid residue at the N-terminus of the membrane protein portion are bound by peptide bonds.

In the polypeptide molecule of Configuration Example 6, an amino acid residue at the C-terminus of the first membrane protein portion and an amino acid residue at the N-terminus of the first peptide linker are bound by a peptide bond. Further, an amino acid residue at the C-terminus of the first peptide linker and an amino acid residue at the N-terminus of the ALP portion are bound by a peptide bond. Further, an amino acid residue at the C-terminus of the ALP portion and an amino acid residue at the N-terminus of the second peptide linker are bound by a peptide bond. Further, an amino acid residue at the C-terminus of the second peptide linker and an amino acid residue at the N-terminus of the second membrane protein portion are bound by peptide bonds.

The amino acid sequence of the polypeptide molecule of the present embodiment may contain a signal sequence as long as it is necessary for production of the polypeptide molecule of the present embodiment. In the present specification, a polypeptide chain represented by a signal sequence is referred to as "signal peptide". The signal peptide is preferably a secretion signal peptide possessed by a secreted protein secreted extracellular, for example. The secretion signal peptide is known per se, and examples thereof include a signal peptide of luciferase (MKTLILAVALVYCATVHC: SEQ ID NO: 14). The secretion signal peptide is preferably added to the N-terminus of the polypeptide molecule. That is, the amino acid residue at the C-terminus of the amino acid sequence of the secretion signal peptide may be followed by the amino acid residue at the N-terminus of the amino acid sequence of the polypeptide molecule of the present embodiment.

The amino acid sequence of the polypeptide molecule of the present embodiment may include an amino acid sequence of a peptide tag, if necessary for production of the polypeptide molecule of the present embodiment. Examples of the peptide tag include known tags used for purification of recombinant protein. Examples of the tag include a Spot-Tag (registered trademark) (PDRVRAVSHWSS: SEQ ID NO: 15), a glutathione S-transferase (GST) tag, a histidine tag, and the like. The peptide tag may be added to either the N-terminus or the C-terminus of the polypeptide molecule. That is, the amino acid residue at the C-terminus of the amino acid sequence of the peptide tag may be followed by the amino acid residue at the N-terminus of the amino acid sequence of the polypeptide molecule of the present embodiment. Alternatively, the amino acid residue at the C-terminus of the amino acid sequence of the polypeptide molecule of the present embodiment may be followed by the amino acid residue at the N-terminus of the amino acid sequence of the peptide tag.

The calibrator of the present embodiment includes the polypeptide molecule of the present embodiment, and is used for measuring the activity or concentration of ALP contained in EV in a sample. The calibrator of the present embodiment can be said to be a reagent containing the polypeptide molecule of the present embodiment. The calibrator of the present embodiment may be in a form of solid (for example, powder, crystal, freeze-dried product, or the like) or in a form of liquid (for example, solution, suspension, emulsion, or the like). When the calibrator is a solid, it is usually dissolved in a solvent and used. The solvent is preferably an aqueous solvent, and examples thereof include physiological saline solutions, buffer solutions, and the like. The buffer solution is a buffer solution having a buffering effect at a pH near neutrality (for example, a pH of 6 or more and 8 or less). Examples of the buffer solution include Good buffers such as MES, HEPES, PIPES, tris buffered saline (TBS), phosphate buffered saline (PBS), and the like.

The calibrator of the present embodiment may contain additives. Examples of the additive include protein stabilizers such as bovine serum albumin (BSA) and sodium caseinate, preservatives such as sodium azide, and inorganic salts such as sodium chloride.

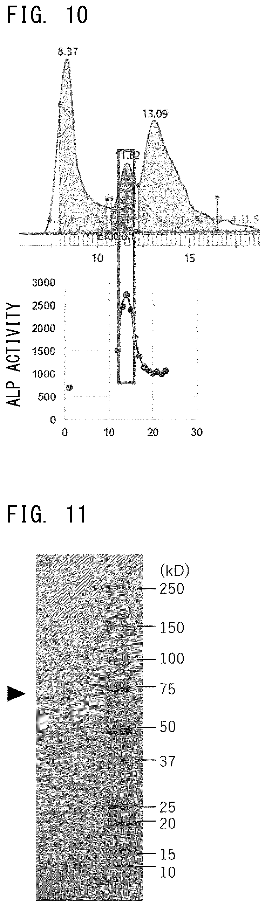

The calibrator of the present embodiment may be in the form of one reagent. The calibrator in the form of one reagent may be properly diluted with the aqueous solvent described above and used. As a result, a plurality of calibrators containing the polypeptide molecules of the present embodiment at different concentrations can be prepared. An example of the calibrator of the present embodiment in the form of one reagent is shown in Fig. 2. In Fig. 2, 10 denotes a kit including a calibrator. 11 denotes a first container containing the calibrator containing the polypeptide molecule of the present embodiment. 12 denotes a packing box. 13 denotes an attached document. The attached document may describe a method for using the calibrator of the present embodiment, a storage method, a composition, and the like.

The calibrator of the present embodiment may be in a form of a reagent set. The form of the reagent set refers to providing a plurality of calibrators (hereinafter, also referred to as a "set of calibrators") containing the polypeptide molecules of the present embodiment at different concentrations. The number of calibrators included in the set of calibrators can be selected from, but not limited to, 2, 3, 4, 5, 6, and 7, for example. Concentration of the polypeptide molecule of the present embodiment in each calibrator is not particularly limited, and is preferably set so as to be able to prepare a calibration curve described later. The calibrator having the highest concentration of polypeptide molecules of the present embodiment may contain the polypeptide molecule of the present embodiment at a concentration of 2 times or more and 1000 times or less as large as that of the calibrator having the lowest concentration. The set of calibrators may contain, as a control, an aqueous solvent containing no polypeptide molecule of the present embodiment.

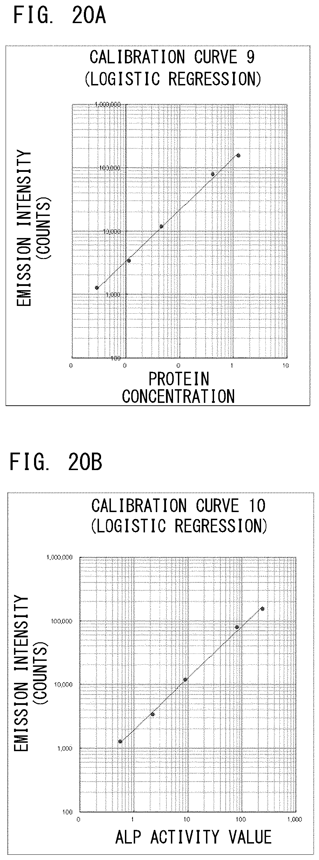

An example of the calibrator of the present embodiment in the form of a reagent set is shown in Fig. 3A. In Fig. 3A, 20 denotes a kit including a set of calibrators. 21 denotes a first container containing the first calibrator containing the polypeptide molecule of the present embodiment. 22 denotes a second container containing the second calibrator containing the polypeptide molecule of the present embodiment at a concentration different from that of the first calibrator. 23 denotes a packing box. 24 denotes an attached document.

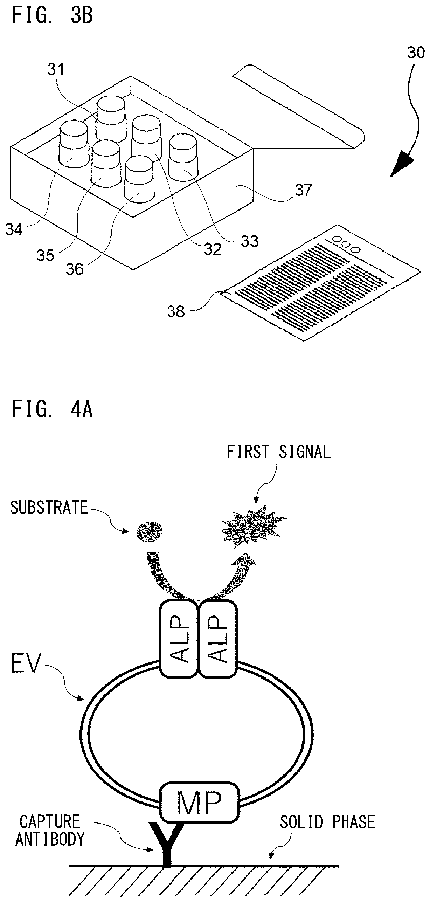

Another example of the calibrator of the present embodiment in the form of a reagent set is shown in Fig. 3B. In Fig. 3B, 30 denotes a kit including a set of calibrators. 31 denotes a first container containing the first calibrator containing the polypeptide molecule of the present embodiment. 32 denotes a second container containing a second calibrator containing the polypeptide molecule of the present embodiment at a concentration different from that of the first calibrator. 33 denotes a third container containing the third calibrator containing the polypeptide molecule of the present embodiment at a concentration different from that of the second calibrator. Reference numeral 34 denotes a fourth container containing a fourth calibrator containing the polypeptide molecule of the present embodiment at a concentration different from the third calibrator. 35 denotes a fifth container containing the fifth calibrator containing the polypeptide molecule of the present embodiment at a concentration different from that of the fourth calibrator. 36 denotes a sixth container containing the aqueous solvent containing no polypeptide molecule of the present embodiment. 37 denotes a packing box. 38 denotes an attached document.

The kit including the set of calibrators may further include a container containing the capture antibody, a container containing the solid phase, and a container containing a substrate of ALP. In this case, the kit including the set of calibrators is also a reagent kit for measuring the activity of ALP contained in EV in a sample. Alternatively, the kit including the set of calibrators may further include a container containing the capture antibody, a container containing the detection antibody, and a container containing the solid phase. In this case, the kit including the set of calibrators is also a reagent kit for measuring the concentration of ALP contained in EV in a sample.

The capture antibody, the solid phase, the substrate of ALP, the detection antibody, and the labeling substance used in the measurement method of the present embodiment will be described. The capture antibody refers to an antibody that specifically binds to a test substance, and is used for capturing the test substance on a solid phase by being immobilized on the solid phase. As used herein, the term "antibody" includes full-length antibodies and fragments thereof. Examples of the fragments of antibody include Fab, Fab', F(ab')2, Fd, Fd', Fv, light chain, heavy chain variable region (VHH) of heavy chain antibody, reduced IgG (rIgG), single chain antibody (scFv), and the like. In the measurement method of the present embodiment, the capture antibody is an antibody that specifically binds to a membrane protein contained in EV. When the polypeptide molecule of the present embodiment has the same type of membrane protein portion as the membrane protein present in the EV containing ALP, the capture antibody can also bind to the polypeptide molecule of the present embodiment.

Examples of the capture antibody include an anti-CD9 antibody, an anti-CD63 antibody, an anti-CD81 antibody, an anti-Annexin A1 antibody, an anti-Annexin A6 antibody, an anti-Pit 1 antibody, and the like. The capture antibody may be either a monoclonal antibody or a polyclonal antibody, but is preferably a monoclonal antibody. Examples of the monoclonal antibody clone include H19a (anti-CD9 antibody), 12A12 (anti-CD9 antibody), H5C6 (anti-CD63 antibody), 5A6 (anti-CD81 antibody), EH17a (anti-Annexin A1 antibody), E-5 (anti-Annexin A6), 6A9-F2 (anti-Pit 1 antibody), and the like. These monoclonal antibodies are commercially available.

The solid phase may be any insoluble carrier capable of immobilizing the capture antibody. Material for composing the solid phase is selectable, without special limitation, typically from organic polymer compound, inorganic compound, and biopolymer. The organic polymer compound is exemplified by latex, polystyrene and polypropylene. The inorganic compound is exemplified by magnetic substance (iron oxide, chromium oxide, ferrite, etc.), silica, alumina and glass. The biopolymer is exemplified by insoluble agarose, insoluble dextran, gelatin and cellulose. Two or more of them may be used in combination. The solid phase may have any form not specifically limited, and is exemplified by particle, microplate, microtube, test tube and membrane. Among them, particles (particularly magnetic particles) and microplate are preferable.

Embodiments of immobilization of the capture antibody on the solid phase are not specifically limited. For example, the capture antibody and the solid phase may be directly bound, or the capture antibody and the solid phase may be indirectly bound via another substance. Examples of the direct binding between the solid phase and the capture antibody include adsorption or covalent binding to the surface of the solid phase by hydrophobic interaction. For example, when the solid phase is an ELISA microplate, the capture antibody is immobilized in a well of the plate by adsorption. When the solid phase has a functional group on the surface, the capture antibody can be immobilized on the surface of the solid phase by covalent binding using the functional group. For example, when the solid phase is a particle having a carboxy group, a carboxy group on the particle surface is activated with WSC and then reacted with NHS to form an NHS ester. Then, when the particle having the NHS ester is contacted with the capture antibody, the NHS ester reacts with an amino group of the capture antibody, and the capture antibody is covalently immobilized on the particle surface.

Examples of the indirect binding between the solid phase and the capture antibody include binding via a molecule that specifically binds to the capture antibody. By previously immobilizing such a molecule on the surface of the solid phase, the capture antibody can be immobilized on the solid phase. Examples of the molecule that specifically binds to the capture antibody include protein A, protein G, and the like. A combination of substances interposed between the capture antibody and the solid phase can be used to bind them. Examples of the combination of substances include combinations of any of biotin and its analogs and any of biotin-binding sites, a hapten and an anti-hapten antibody and the like. The biotin and its analogs include biotin and biotin analogs such as desthiobiotin. The biotin-binding sites include avidin and avidin analogs such as streptavidin and tamavidin (registered trademark). For example, when the capture antibody is modified with biotin, the capture antibody can be immobilized on a solid phase on which avidins are immobilized.

As a substrate of ALP, chemiluminescent substrate such as CDP-Star (registered trademark) (4-chloro-3-(methoxyspiro[1,2-dioxetan-3,2'-chloro]decan]-4-yl)phenyl phosphate, CSPD (3-(4-methoxyspiro[1,2-dioxetan-3,2'-chloro)tricyclo[3.1.1 $^{3,7}$]decan]-4-yl)phenyl phosphate Examples thereof include chromogenic substrates such as 5-bromo-4-chloro-3-indolylphosphate (BCIP), 2-sodium 5-bromo-6-chloro-indolylphosphate, and p-nitrophenylphosphate. Among them, CDP-Star (registered trademark) is preferably used.

The detection antibody refers to an antibody that specifically binds to a test substance, and provides a detectable signal via a labeling substance. The detection antibody is preferably not immobilized on the solid phase. In the measurement method of the present embodiment, the detection antibody is an antibody that specifically binds to ALP contained in EV. When the polypeptide molecule of the present embodiment has the same type of ALP moiety as the ALP present in EV, the detection antibody can also bind to the polypeptide molecule of the present embodiment. Examples of the detection antibody include an anti-TNAP antibody, an anti-IAP antibody, and the like. The detection antibody may be either a monoclonal antibody or a polyclonal antibody, but is preferably a monoclonal antibody.

The detection antibody preferably contains a labeling substance. For example, the detection antibody and the labeling substance may be directly bound, or the detection antibody and the labeling substance may be indirectly bound via another substance. Examples of the direct binding between the detection antibody and the labeling substance include covalent binding of the detection antibody and the labeling substance using a commercially available crosslinker or labeling kit. The antibody to which the labeling substance is covalently bound is also referred to as a labeled antibody. Examples of the indirect binding between the detection antibody and the labeling substance include binding between the detection antibody and a labeled antibody (labeled secondary antibody) that specifically binds to the detection antibody.

The labeling substance is not particularly limited, and examples thereof include a substance that itself generates a signal (hereinafter, also referred to as "signal generating substance"), a substance that catalyzes a reaction of another substance to generate a signal, and the like. Examples of the signal generating substance include a fluorescent substance, a radioisotope, and the like. Examples of the substance that catalyzes the reaction of another substance to generate a detectable signal include an enzyme. Examples of the enzyme include ALP, peroxidase, β-galactosidase, and luciferase. Examples of the fluorescent substance include fluorescent dyes such as fluorescein isothiocyanate (FITC), rhodamine, and Alexa Fluor (registered trademark), and fluorescent proteins such as GFP. Examples of the radioisotope include $^{125}$I, $^{14}$C, $^{32}$P, and the like. The labeling substance is preferably an enzyme, and ALP and peroxidase are particularly preferable. The ALP as a labeling substance can be used in a case where a signal derived from the ALP portion of the ALP and the polypeptide molecule contained in EV is not generated by using a buffer containing an ALP inhibitor such as levamisole in the formation of the first complex and the second complex described later.

In the measurement method of the present embodiment, as described above, the activity value or concentration of ALP contained in EV in a sample is acquired using the calibrator of the present embodiment. The sample is not specifically limited as long as EV can be contained. A preferred sample is a biological sample. The biological sample is exemplified by blood (whole blood), plasma, serum, urine, lymph, tissue fluid, cerebrospinal fluid and saliva. Among them, blood, plasma, and serum are preferable. When the sample contains insoluble impurities such as cells, the impurities may be removed from the sample by known means such as centrifugation or filtration. The sample may be diluted with a suitable aqueous medium as necessary. Such aqueous medium is not particularly limited as long as it does not interfere with the measurement described later, and examples thereof include water, physiological saline, buffer solutions, and the like. The buffer solution is as described above. When plasma is used as the sample, an anticoagulant may be used at the time of blood collection. Examples of the anticoagulant include potassium salt of EDTA, sodium salt of EDTA, sodium citrate, and heparin salt.

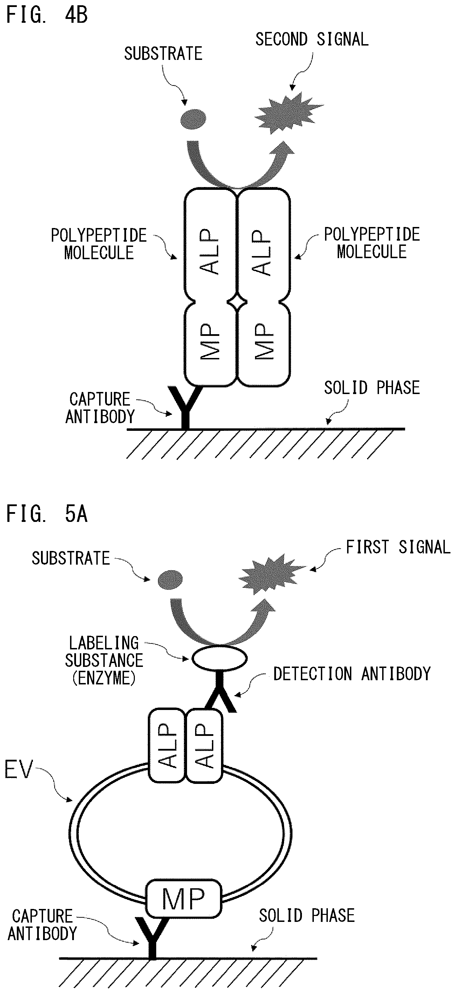

The activity value of ALP contained in EV in the sample can be acquired on the basis of the measured results of the activity of ALP contained in EV in the sample and the ALP activity of the polypeptide molecule in the calibrator of the present embodiment, respectively. The activity value of ALP refers to a value represented by an enzyme unit (also referred to as a unit). One unit (U) refers to the amount of enzyme that acts on 1 μmol of a substrate per minute under optimal conditions. For example, when the activity value of ALP contained in EV in a sample is 1 U/L, there is EV containing ALP in an amount of enzyme that acts on 1 μmol of a substrate per minute in 1 L of the sample. With reference to Figs. 4A and B, an example in which the activity value of ALP contained in EV in a sample is acquired by immunoassay using a capture antibody that specifically binds to a membrane protein contained in EV and a substrate of ALP will be described. The present invention is, however, not limited to this example. Types of immunoassay are not particularly limited, and can be selected from known methods such as enzyme-linked immunosorbent method (ELISA), immunoprecipitation method, and immune complex transfer method (see Japanese Patent Publication H1-254868, which is incorporated by reference). The immunoassay may be performed by a commercially available automatic immunoassay device such as a series of HISCL (registered trademark) (Sysmex Corporation) and HI-1000 (Sysmex Corporation).

First, as shown in Fig. 4A, a first complex containing EV in a sample and a capture antibody that specifically binds to a membrane protein contained in EV is formed on a solid phase. In the EV in the figure, "MP" is a membrane protein present on a surface of EV, and "ALP" is ALP present on a surface of EV. In the figure, ALP contained in EV is shown as a homodimer, but this is because ALP as an enzyme in a living body or a biological sample usually forms a homodimer. The first complex is formed by mixing a sample containing EV and a solution of the capture antibody, whereby the capture antibody is bound to a membrane protein present on a surface of EV. Then, the solution containing the first complex is contacted with the solid phase capable of immobilizing the capture antibody, thereby forming the first complex on the solid phase. Alternatively, a solid phase in which the capture antibody is immobilized in advance may be used. That is, the first complex can be formed on the solid phase by contacting the sample containing EV with the solid phase immobilized with the capture antibody. Then, as shown in Fig. 4A, EV in the first complex formed on the solid phase is contacted with the substrate of ALP. This contact reacts the ALP contained in the EV in the first complex with the substrate to generate a signal. This signal is referred to as a first signal. By measuring this first signal, the activity of ALP contained in EV in the sample is measured. As used herein, the phrase "measuring a signal" includes quantifying a signal intensity and semi-quantitatively detecting the signal intensity. The phrase "semi-quantitatively detecting the signal intensity" means to detect the signal intensity in plural stages such as "no signal generated", "weak", "strong", and the like. In the measurement of the signal, it is preferable to quantify the intensity of the signal.

Next, as shown in Fig. 4B, a second complex comprising the polypeptide molecule in the calibrator of the present embodiment and the capture antibody described above is formed on a solid phase. In the polypeptide molecule in the figure, the "MP" is a membrane protein portion, and the "ALP" is an ALP portion. In Fig. 4B, the polypeptide molecule forms a homodimer. This is because, as described above, the polypeptide molecule of the present embodiment is considered to exert ALP activity by forming a homodimer via an ALP moiety. The second complex is formed by mixing the calibrator of the present embodiment and a solution of the capture antibody, so that the capture antibody is bound to a membrane protein portion of the polypeptide molecule. Now, the capture antibody used for forming the second complex is the same as the capture antibody used for forming the first complex. Then, the solution containing the second complex is contacted with the solid phase to form the second complex on the solid phase. Alternatively, a solid phase in which the capture antibody is immobilized in advance may be used. That is, the second complex can be formed on the solid phase by contacting the calibrator of the present embodiment with the solid phase immobilized with the capture antibody. Then, as shown in Fig. 4B, the polypeptide molecule in the second complex formed on the solid phase is contacted with the substrate of ALP. This contact reacts the ALP portion of the polypeptide molecule in the second complex with the substrate to generate a signal. This signal is referred to as a second signal. By measuring this second signal, the ALP activity of the polypeptide molecule in the calibrator of the present embodiment is measured.

Now, the concentration of polypeptide molecules and the ALP activity value contained in the calibrator of the present embodiment are known. The concentration of the polypeptide molecule of the present embodiment refers to protein content (unit is μg/mL, for example). The concentration of the polypeptide molecule can be acquired by, for example, a known protein quantification method such as absorbance measurement. The ALP activity value of the polypeptide molecule of the present embodiment refers to an activity value (U/L) when the polypeptide molecule is regarded as ALP. The ALP activity value of the polypeptide molecule of the present embodiment can be acquired by a biochemical measurement method of ALP. In the biochemical measurement method, ALP and a substrate are directly reacted to obtain an activity value (U/L) of ALP. Since the calibrator of the present embodiment does not contain a substance having ALP activity in addition to the polypeptide molecule of the present embodiment, the ALP activity value of the polypeptide molecule can be acquired by biochemical measurement. For example, p-nitrophenyl phosphate as a substrate and the calibrator of the present embodiment are mixed, and p-nitrophenol generated by hydrolyzing the substrate by an ALP portion of a polypeptide molecule is optically measured. Therefore, with regard to the calibrator of the present embodiment, it is clear that the measured value of the second signal is related to the concentration as protein and the activity value as ALP. From this, the activity value or concentration of ALP contained in EV in the sample is acquired from the measured value of the first signal and the measured value of the second signal.

Preferably, a plurality of calibrators having different concentrations of the polypeptide molecules of the present embodiment are measured to obtain measured values of the second signals of the calibrators. A calibration curve can be prepared by acquiring the measured values of the second signals from a plurality of calibrators. The calibration curve can be prepared, for example, by plotting the measured values of the second signals acquired from a plurality of calibrators on an XY plane in which the concentration of the polypeptide molecule or the activity value of ALP is taken on an X-axis and the measured values of the signals are taken on a Y-axis to obtain a straight line or a curve by a known method such as a least squares method. By applying the measured value of the first signal to this calibration curve, the concentration of ALP or the activity value of ALP contained in EV in the sample can be acquired. A regression equation representing the obtained calibration curve may be acquired. By the obtained regression formula, the measured value of the first signal can be converted into the activity value or concentration of ALP contained in EV in a sample.

In the examples illustrated in Figs. 4A and B, the calibrator is measured after the sample is measured, but the order of measurement is not specifically limited. The sample may be measured after the calibrator is measured. Alternatively, the sample and the calibrator may be measured in parallel.

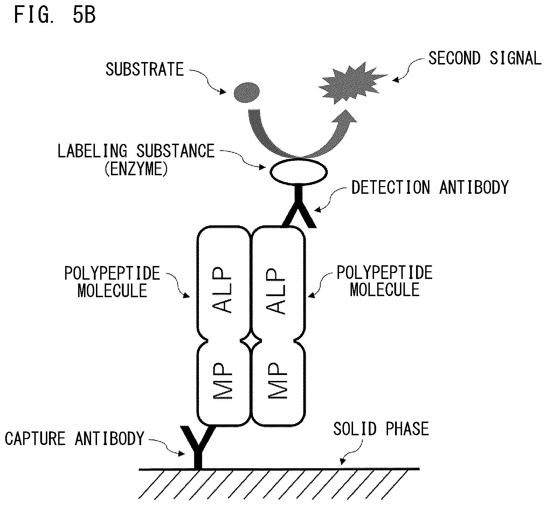

In a further embodiment, the concentration of ALP contained in EV in a sample can be acquired on the basis of detection results of ALP contained in EV in a sample and polypeptide molecules in the calibrator of the present embodiment, each detected. With reference to Figs. 5A and B, an example in which the concentration of ALP contained in EV in a sample is acquired by immunoassay using a capture antibody that specifically binds to a membrane protein contained in EV and a detection antibody that specifically binds to ALP contained in EV will be described. The present invention is, however, not limited to this example.

First, as shown in Fig. 5A, a first complex comprising EV in a sample, a capture antibody that specifically binds to a membrane protein contained in EV, a detection antibody that specifically binds to ALP contained in EV, and a labeling substance is formed on a solid phase. "MP" and "ALP" in the figure are the same as those in Fig. 4A. In the figure, ALP contained in EV is shown as a homodimer similarly to Fig. 4A. In the figure, a labeling substance is bound to a detection antibody. The labeling substance is an enzyme. The first complex is formed by mixing a sample containing EV, a solution of the capture antibody, and a solution of the detection antibody containing a labeling substance, whereby the capture antibody and the detection antibody are bound to the membrane protein and ALP present on the surface of EV, respectively. Then, the solution containing the first complex is contacted with the solid phase capable of immobilizing the capture antibody, thereby forming the first complex on the solid phase. Alternatively, a solid phase in which the capture antibody is immobilized in advance may be used. That is, the first complex can be formed on the solid phase by contacting the sample containing EV, the solid phase having the capture antibody immobilized thereon, and the detection antibody that contains a labeling substance. Then, as shown in Fig. 5A, the EV in the first complex formed on the solid phase is contacted with the substrate of the labeling substance. This contact reacts the labeling substance contained in the first complex with the substrate to generate a signal. This signal is referred to as a first signal. Since the first signal is generated from the detection antibody bound to ALP contained in EV and the labeling substance, ALP contained in EV in the sample is detected by measuring the first signal.

Next, as shown in Fig. 5B, a polypeptide molecule in the calibrator of the present embodiment, the capture antibody, the detection antibody, and a second complex containing the labeling substance are formed on a solid phase. "MP" and "ALP" in the figure are the same as those in Fig. 4B. In the figure, the polypeptide molecule is shown as a homodimer similarly to Fig. 4B. However, when the detection antibody is used, the ALP activity of the polypeptide molecule itself in the calibrator is not measured, and thus the polypeptide molecule may be a monomer. The second complex is formed by mixing the calibrator of the present embodiment, a solution of the capture antibody, and a solution of the detection antibody containing a labeling substance, whereby the capture antibody and the detection antibody are bound to the membrane protein and ALP present on the surface of EV, respectively. Now, the capture antibody, the detection antibody, and the labeling substance used for forming the second complex are the same as the capture antibody, the detection antibody, and the labeling substance used for forming the first complex. Then, the solution containing the second complex is contacted with the solid phase to form the second complex on the solid phase. Alternatively, a solid phase in which the capture antibody is immobilized in advance may be used. That is, the second complex can be formed on the solid phase by contacting the calibrator of the present embodiment, the solid phase having the capture antibody immobilized thereon, and the detection antibody that contains a labeling substance. Then, as shown in Fig. 5B, the polypeptide molecule in the second complex formed on the solid phase is contacted with the substrate of the labeling substance. This contact reacts the labeling substance contained in the second complex with the substrate to generate a signal. This signal is referred to as a second signal. Since the second signal originates from the detection antibody bound to the ALP portion of the calibrator and the labeling substance, ALP of the polypeptide molecule in the calibrator of the present embodiment is detected by measuring the second signal.

As described above, the value indicating the concentration (protein content) of the polypeptide molecule contained in the calibrator of the present embodiment is known. Therefore, with regard to the calibrator of the present embodiment, it is apparent that the measured value of the second signal is related to the protein content. From this, the concentration of ALP contained in EV in the sample is acquired from the measured values of the first signal and the measured values of the second signal. Preferably, a plurality of calibrators having different concentrations of the polypeptide molecules of the present embodiment are measured to obtain measured values of the second signals of the calibrators. A calibration curve can be prepared by acquiring the measured values of the second signals from a plurality of calibrators. The calibration curve can be prepared, for example, by plotting the measured values of the second signals acquired from a plurality of calibrators on an XY plane in which the concentration of the polypeptide molecule is taken on an X-axis and the measured values of the signals are taken on a Y-axis to obtain a straight line or a curve by a known method such as a least squares method. By applying the measured value of the first signal to the calibration curve, the concentration of ALP contained in EV in the sample can be acquired. A regression equation representing the obtained calibration curve may be acquired. By the obtained regression equation, the measured value of the first signal can be converted into the concentration of ALP contained in EV in the sample.

In the examples illustrated in Figs. 5A and B, the calibrator is measured after the sample is measured, but the order of measurement is not specifically limited. The sample may be measured after the calibrator is measured. Alternatively, the sample and the calibrator may be measured in parallel.

In the measurement method of the present embodiment, B/F (Bound/Free) separation for removing unreacted free components not forming a complex may be performed between formation of the complex and measurement of the signal. The unreacted free component refers to a component not constituting a complex. Examples thereof include a capture antibody and a detection antibody that do not bind to an EV or a polypeptide molecule. The means of B/F separation is not particularly limited, and when the solid phase is a particle, B/F separation can be performed by recovering only the solid phase capturing the complex by centrifugation. When the solid phase is a container such as a microplate or a microtube, B/F separation can be performed by removing a liquid containing an unreacted free component. When the solid phase is a magnetic particle, B/F separation can be performed by aspirating and removing a liquid containing an unreacted free component by a nozzle while magnetically constraining the magnetic particles with a magnet, which is preferable from the viewpoint of automation. After removing the unreacted free component, the solid phase capturing the complex may be washed with a suitable aqueous medium such as PBS.

A method for producing the polypeptide molecule of the present embodiment (hereinafter, also referred to as "method for producing the present embodiment") will be described. In the production method of the present embodiment, an expression vector containing a gene encoding the polypeptide molecule of the present embodiment is used. The polypeptide molecule of the present embodiment is expressed by introducing the expression vector into a cell and culturing the cell. Then, the polypeptide molecule of the present embodiment expressed by the cell is acquired.

The gene encoding the polypeptide molecule of the present embodiment is a series of polynucleotide fragments including a gene encoding an amino acid sequence of ALP (also referred to as an ALP gene) and a gene encoding an amino acid sequence of a membrane protein (also referred to as a membrane protein gene). The ALP gene and the membrane protein gene may be cloned from genomic DNA by a conventional method, or may be synthesized on the basis of known amino acid sequences or base sequences. By incorporating these genes into one expression vector so that the ALP gene and the membrane protein gene become a series of polynucleotide fragments, an expression vector containing a gene encoding the polypeptide molecule of the present embodiment can be acquired.

The type of expression vector is not particularly limited as long as it has a promoter capable of protein expression in a host cell and can insert a desired gene downstream of the promoter. Examples of the expression vector include a plasmid vector, a viral vector, and the like. A commercially available expression vector may be used. The expression vector may optionally contain genes or polynucleotide fragments other than the ALP gene and the membrane protein gene. Examples of the gene and the polynucleotide fragment include a gene encoding a peptide linker (also referred to as a linker gene), a gene encoding a signal peptide, a gene encoding a peptide tag, a drug resistance gene, a Kozak sequence, and the like.

The cell is not particularly limited as long as it can be used as a recombinant expression system, and examples thereof include mammalian cells, insect cells, plant cells, yeast, and E. coli. The expression vector containing the gene encoding the polypeptide molecule of the present embodiment can be transformed or transfected into a cell to obtain a cell containing the expression vector. The transformation and the transfection may be performed by known methods depending on the type of the expression vector. Examples of the method include lipofection method, calcium phosphate method, and electroporation method. A commercially available kit such as Expi293 (trademark) Expression System, ExpiCHO (trademark) Expression System (Thermofisher Scientific) may be used.

In the expression vector, the ALP gene may be integrated either upstream or downstream of the membrane protein gene. When the ALP gene is integrated upstream of the membrane protein gene, a polypeptide molecule in which an amino acid residue at the C-terminus of the ALP portion and an amino acid residue at the N-terminus of the membrane protein portion are bound by peptide binding is expressed. When the ALP gene is integrated downstream of the membrane protein gene, a polypeptide molecule in which an amino acid residue at the C-terminus of the membrane protein portion and an amino acid residue at the N-terminus of the ALP portion are bound by peptide binding is expressed. In the expression vector, a linker gene may be inserted between the ALP gene and the membrane protein gene. This expresses a polypeptide molecule in which the ALP portion and the membrane protein portion are bound via a peptide linker.

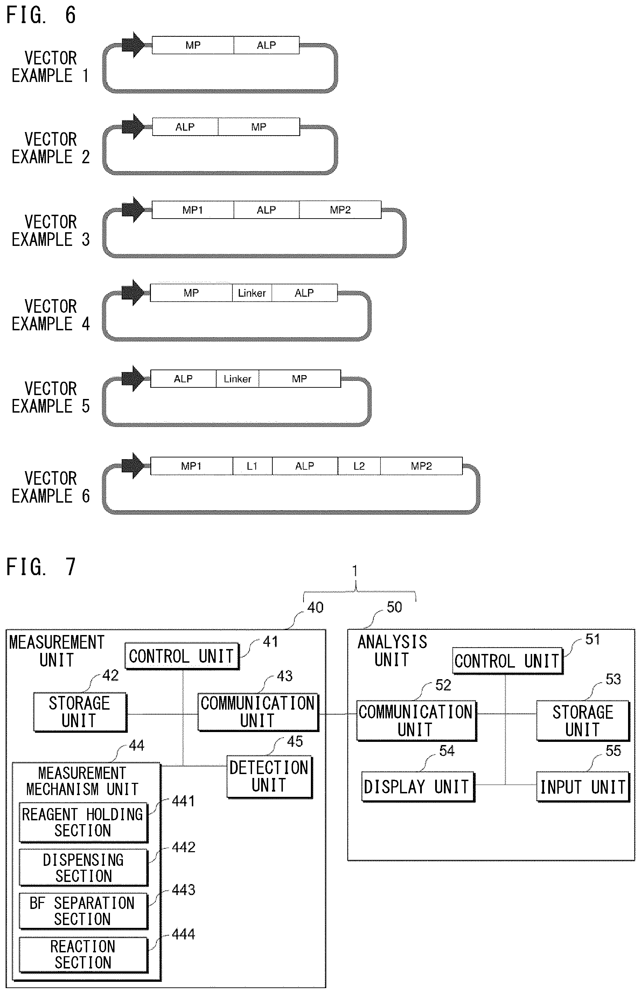

An example of an expression vector containing an ALP gene and a membrane protein gene will be described with reference to Fig. 6. However, the expression vector used in the production method of the present embodiment is not limited to these examples. In the figure, the expression vector is exemplified as plasmid DNA, and an arrow denotes a promoter. "MP" represents a membrane protein gene, "MP1" represents a first membrane protein gene, "MP2" represents a second membrane protein gene, "ALP" represents an ALP gene, "Linker" represents a linker gene, "L1" represents a first linker gene, and "L2" represents a second linker gene. The first membrane protein gene and the second membrane protein gene may be the same or different. The first linker gene and the second linker gene may be the same or different.

The expression vector of vector example 1 contains a membrane protein gene and an ALP gene in this order downstream of a promoter. That is, in the polynucleotide fragment incorporated in the expression vector, the nucleotide at the 3' end of the membrane protein gene and the nucleotide at the 5' end of the ALP gene are bound by a phosphodiester bond. By introducing the expression vector of vector example 1 into the cell, the polypeptide molecule of configuration example 1 shown in Fig. 1A is expressed.

The expression vector of vector example 2 contains the ALP gene and the membrane protein gene in this order downstream of the promoter. That is, in the polynucleotide fragment incorporated in the expression vector, the nucleotide at the 3' end of the ALP gene and the nucleotide at the 5' end of the membrane protein gene are bound by a phosphodiester bond. By introducing the expression vector of vector example 2 into the cell, the polypeptide molecule of configuration example 2 shown in Fig. 1A is expressed.

The expression vector of vector example 3 contains a first membrane protein gene, an ALP gene, and a second membrane protein gene in this order, downstream of the promoter. That is, in the polynucleotide fragment incorporated in the expression vector, the nucleotide at the 3' end of the first membrane protein gene and the nucleotide at the 5' end of the ALP gene are bound by a phosphodiester bond. Further, the nucleotide at the 3' end of the ALP gene and the nucleotide at the 5' end of the second membrane protein gene are bound by a phosphodiester bond. By introducing the expression vector of vector example 3 into the cell, the polypeptide molecule of configuration example 3 shown in Fig. 1A is expressed.

The expression vector of vector example 4 contains a membrane protein gene, a linker gene, and an ALP gene in this order downstream of a promoter. That is, in the polynucleotide fragment incorporated in the expression vector, the nucleotide at the 3' end of the membrane protein gene and the nucleotide at the 5' end of the linker gene are bound by a phosphodiester bond. Further, the nucleotide at the 3' end of the linker gene and the nucleotide at the 5' end of the ALP gene are bound by a phosphodiester bond. By introducing the expression vector of vector example 4 into the cell, the polypeptide molecule of configuration example 4 shown in Fig. 1B is expressed.

The expression vector of vector example 5 contains, downstream of the promoter, an ALP gene, a linker gene, and a membrane protein gene in this order. That is, in the polynucleotide fragment incorporated in the expression vector, the nucleotide at the 3' end of the ALP gene and the nucleotide at the 5' end of the linker gene are bound by a phosphodiester bond. Further, the nucleotide at the 3' end of the linker gene and the nucleotide at the 5' end of the membrane protein gene are bound by a phosphodiester bond. By introducing the expression vector of vector example 5 into the cell, the polypeptide molecule of configuration example 5 shown in Fig. 1B is expressed.

The expression vector of vector example 6 contains a first membrane protein gene, a first linker gene, an ALP gene, a second linker gene, and a second membrane protein gene in this order, downstream of the promoter. That is, in the polynucleotide fragment incorporated in the expression vector, the nucleotide at the 3' end of the first membrane protein gene and the nucleotide at the 5' end of the first linker gene are bound by a phosphodiester bond. Further, the nucleotide at the 3' end of the first linker gene and the nucleotide at the 5' end of the ALP gene are bound by a phosphodiester bond. Further, the nucleotide at the 3' end of the ALP gene and the nucleotide at the 5' end of the second linker gene are bound by a phosphodiester bond. Furthermore, the nucleotide at the 3' end of the second linker gene and the nucleotide at the 5' end of the second membrane protein gene are bound by a phosphodiester bond. By introducing the expression vector of vector example 6 into the cell, the polypeptide molecule of configuration example 6 shown in Fig. 1B is expressed.

In the production method of the present embodiment, a cell containing the expression vector described above is cultured to cause the cell to express the polypeptide molecule of the present embodiment. The method for culturing a cell is known per se, and can be appropriately determined according to the type of the cell and the expression vector and the method for introducing an expression vector. For example, a cell including the expression vector described above may be cultured in a medium suitable for culture of a cell at an appropriate environment (for example, 37° C. in a 5% $CO_2$).

In the production method of the present embodiment, a polypeptide molecule expressed by a cell is acquired. For example, a cell expressing the polypeptide molecule of the present embodiment may be dissolved in a solution containing an appropriate solubilizing agent to recover the polypeptide molecule released in the solution. When the cell secretes the polypeptide molecule of the present embodiment into a medium, the culture supernatant is recovered. The free polypeptide molecule can be recovered by known methods such as size exclusion column chromatography, hydrophobic interaction chromatography, and affinity chromatography. For a solution containing the recovered polypeptide molecule, it is preferable to measure the concentration (protein content) and the ALP activity value (U/L) of the polypeptide molecule. By determining these values, the solution containing the acquired polypeptide molecule can be used as it is or properly diluted, as the calibrator of the present embodiment.

A further embodiment provides a use of a polypeptide molecule for the manufacture of a calibrator used for measuring the activity or concentration of ALP contained in EV in a sample, wherein the polypeptide molecule is a polypeptide molecule having a series of amino acid sequences comprising an amino acid sequence of ALP and an amino acid sequence of a membrane protein present on a surface of EV.

A further embodiment provides an immunoassay device capable of executing the measurement method of the present embodiment. An example of the immunoassay device will be described with reference to the drawings. The present invention is, however, not limited to this example. Referring to Fig. 7, an immunoassay device 1 includes a measurement unit 40 and an analysis unit 50. The measurement unit 40 includes a control unit 41, a storage unit 42, a communication unit 43, a measurement mechanism unit 44, and a detection unit 45.

The control unit 41 includes, for example, a CPU and its peripheral circuit. The control unit 41 controls an operation of each unit of the measurement unit 40 and the measurement mechanism unit 44 according to each function. The storage unit 42 includes a solid state drive (SSD) or a hard disk drive (HDD) that stores various programs, various data, and the like for the control unit 41 to control each unit of the measurement unit 40. The communication unit 43 inputs and outputs data to and from an external device under control of the control unit 41. The communication unit 43 includes an interface circuit using an arbitrary communication standard such as Ethernet (registered trademark) and IEEE 1394, for example.

The measurement mechanism unit 44 mainly includes a reagent holding section 441, a dispensing section 442, a BF separation section 443, and a reaction section 444. The reagent holding section 441 holds a reagent. The dispensing section 442 dispenses the reagent held by the reagent holding section 441 and the sample or calibrator into the reaction container. The BF separation section 443 executes the BF separation processing described above. The reaction section 444 heats the reaction container to promote a reaction between the reagent and the sample or the calibrator. The detection unit 45 detects a signal generated from the first complex and the second complex in the reaction container, and outputs a measured value of a signal corresponding to the signal.

The analysis unit 50 mainly includes a control unit 51, a communication unit 52, a storage unit 53, a display unit 54, and an input unit 55. The control unit 51 controls an operation of each unit of the analysis unit 50 according to each function. The control unit 51 includes, for example, a CPU and its peripheral circuit. The communication unit 52 is a circuit that inputs and outputs data to and from an external device including the communication unit 43 of the measurement unit 40 under control of the control unit 51. The communication unit 52 includes, for example, an interface circuit using an arbitrary communication standard such as Ethernet and IEEE 1394, and the like. The storage unit 53 stores programs executed by the control unit 51, various data, and the like. The storage unit 53 includes an SSD or a hard disk similarly to the storage unit 42.

The display unit 54 is a touch panel type display including a display device such as a liquid crystal display or an organic EL display. The input unit 55 is a device that inputs various commands such as a command for instructing preparation of a calibration curve, various data necessary for operating the immunoassay device 1, and the like to the immunoassay device 1. The input unit 55 includes a keyboard, a pointing device such as a mouse or a touch panel, a plurality of input switches to which predetermined functions are assigned, and the like.

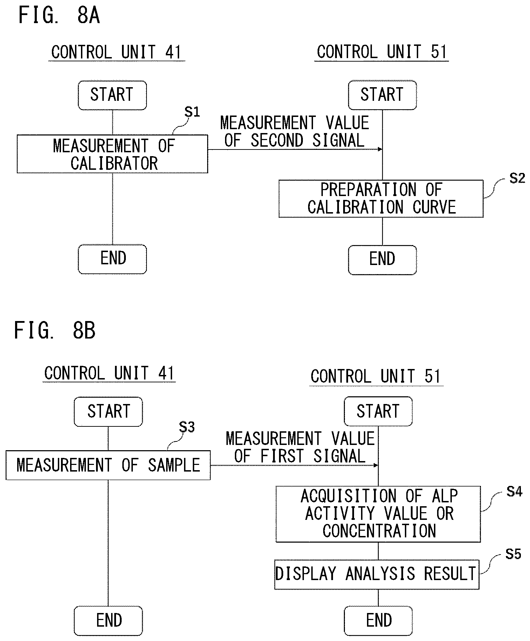

Each step executed by the immunoassay device 1 will be described with reference to Figs. 8A and B. In step S1, the control unit 41 executes measurement of a calibrator. The control unit 41 controls the measurement mechanism unit 44 to form a second complex including the polypeptide molecule contained in the calibrator and the capture antibody on the solid phase, and contact the polypeptide molecule in the second complex on the solid phase with the substrate of ALP. Alternatively, the control unit 41 controls the measurement mechanism unit 44 to form a second complex including the polypeptide molecule contained in the calibrator, the capture antibody, the detection antibody, and the labeling substance (enzyme) on the solid phase, and contact the labeling substance in the second complex on the solid phase with the substrate of the labeling substance.

More specifically, in step S1, the control unit 41 causes the dispensing section 442 to dispense the various reagents and the calibrator held by the reagent holding section 441 into the reaction container. The reagent held by the reagent holding section 441 is, for example, an R1 reagent containing a capture antibody, an R2 reagent containing a solid phase (magnetic particle), an R4 reagent containing a measurement buffer, and an R5 solution containing a chemiluminescent substrate of ALP. First, the control unit 41 causes the dispensing section 442 to dispense the R1 reagent, the R2 reagent, and the calibrator into the reaction container. As a result, a second complex including the polypeptide molecule and the capture antibody is formed on the solid phase. The control unit 41 causes the BF separation section 443 to remove unreacted components in the reaction container. Next, the control unit 41 causes the dispensing section 442 to dispense the R4 reagent and the R5 reagent into the reaction container. This contacts the polypeptide molecule in the second complex on the solid phase with the substrate of ALP. The reaction container is heated by the reaction section 444 for a predetermined time. Next, the control unit 41 causes the detection unit 45 to detect a second signal generated by contact between the ALP portion in the polypeptide molecule and the substrate.

Alternatively, the reagent held by the reagent holding section 441 is, for example, an R1 reagent containing a capture antibody, an R2 reagent containing a solid phase (magnetic particle), an R3 reagent containing a detection antibody bound to a labeling substance (enzyme), an R4 reagent containing a measurement buffer, and an R5 solution containing a chemiluminescent substrate of an enzyme. In this case, the control unit 41 causes the dispensing section 442 to dispense the R1 reagent, the R2 reagent, the R3 reagent, and the calibrator into the reaction container. As a result, a second complex including the polypeptide molecule, the capture antibody, the detection antibody, and the labeling substance is formed on the solid phase. The control unit 41 causes the BF separation section 443 to remove unreacted components in the reaction container. Next, the control unit 41 causes the dispensing section 442 to dispense the R4 reagent and the R5 reagent into the reaction container. This contacts the polypeptide molecule in the second complex on the solid phase with the substrate of the labeling substance. The reaction container is heated by the reaction section 444 for a predetermined time. Next, the control unit 41 causes the detection unit 45 to detect a second signal generated by contact between the labeling substance in the second complex and the substrate.

The detection unit 45 outputs a measured value of the second signal in response to the detected second signal. The control unit 41 stores the output measured value of the second signal in the storage unit 42. The control unit 41 transmits the measured value of the second signal stored in the storage unit 42 to the analysis unit 50 via the communication unit 43. The control unit 51 stores the measured value of the second signal received via the communication unit 52 in the storage unit 53. The measurement in step S1 is repeated by the number of calibrators supplied to the measurement unit 40.

In step S2, the control unit 51 executes preparation of a calibration curve. The control unit 51 prepares a calibration curve based on the measured value of the second signal stored in the storage unit 53. The preparation of the calibration curve is as described above. The control unit 51 stores the prepared calibration curve in the storage unit 53. The control unit 51 may acquire a regression equation representing the prepared calibration curve. The control unit 51 stores the acquired regression equation in the storage unit 53.

In step S3, the control unit 41 executes measurement of a sample. The control unit 41 controls the measurement mechanism unit 44 in the same manner as in step S1 to form a first complex including EV contained in a sample and a capture antibody on a solid phase, and contact the EV in the first complex on the solid phase with a substrate of ALP. Alternatively, the control unit 41 controls the measurement mechanism unit 44 to form a first complex including EV, the capture antibody, the detection antibody, and the labeling substance (enzyme) on the solid phase, and contact the labeling substance in the first complex on the solid phase with the substrate of the labeling substance. The control unit 41 causes the detection unit 45 to detect a first signal generated by contact with the substrate. The detection unit 45 outputs a measured value of the first signal in response to the detected first signal. The control unit 41 stores the output measured value of the first signal in the storage unit 42. The control unit 41 transmits the signal value stored in the storage unit 42 to the analysis unit 50 via the communication unit 43. The control unit 51 stores the measured value of the first signal received via the communication unit 52 in the storage unit 53.

In step S4, the control unit 51 executes acquisition of an activity value or concentration of ALP. The control unit 51 acquires an activity value or a concentration of ALP contained in EV in a sample by applying the measured value of the first signal obtained from the sample to the calibration curve or the regression equation stored in the storage unit 53. The control unit 51 stores the acquired value in the storage unit 53. In step S5, the control unit 51 executes display of an analysis result. The control unit 51 displays the activity value or the concentration of ALP stored in the storage unit 53 on the display unit 54 as a measurement result of the sample.

Hereinbelow, the present invention will be described in detail by examples, but the present invention is not limited to these examples.

EXAMPLES

[Example for Producing Polypeptide Molecule]

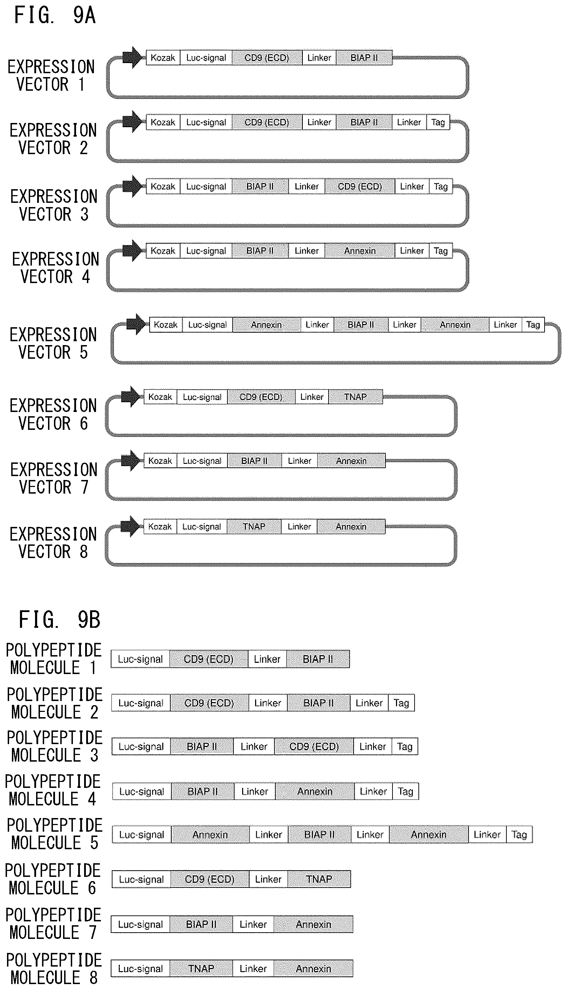

The expression vectors 1 to 8 shown in Fig. 9A were prepared, and the expression vectors were transfected into cells to express the polypeptide molecule. The expressed polypeptide molecule was recovered and purified from the culture supernatant. A schematic diagram of a polypeptide molecule expressed by a cell including the expression vectors 1 to 8 is shown in Fig. 9B. The polypeptide molecules 1 to 8 in Fig. 9B were polypeptide molecules derived from the expression vectors 1 to 8, respectively. Polypeptide molecule 1 was used in Example 1, polypeptide molecules 2 to 5 were used in Example 2, polypeptide molecule 6 was used in Example 3, polypeptide molecule 7 was used in Example 4, and polypeptide molecule 8 was used in Example 5.

(1) Preparation of expression vector

Production of the expression vectors 1 to 8 was commissioned to GeneArt® servicing by Thermofisher Scientific. All expression vectors were prepared by incorporating a synthetic gene encoding a polypeptide molecule downstream of a promoter of a pcDNA (trademark) 3.4-TOPO (registered trademark) vector. A synthetic gene encoding a polypeptide molecule was prepared by a PCR method using an artificially synthesized oligonucleotide based on an amino acid sequence of the polypeptide molecule.

The configuration of the synthetic genes contained in each expression vector was as shown in Fig. 9A. In Fig. 9A, an arrow denotes a promoter, a "Luc-signal" denotes a gene encoding a luciferase signal peptide (SEQ ID NO: 14), a gene encoding an extracellular domain (ECD) (SEQ ID NO: 3), a "Linker" denotes a gene encoding a GS1 peptide linker (SEQ ID NO: 8), a "BIAP II" denotes a gene encoding BIAP II (SEQ ID NO: 1), and "Tag" denotes a gene encoding Spot-Tag (SEQ ID NO: 15)."Annexin" denotes a gene encoding an epitope region of Annexin A1 (SEQ ID NO: 7), and "TNAP" denotes a gene encoding full-length human tissue non-specific ALP (SEQ ID NO: 2).

The Kozak sequence (5'-CACC-3') was introduced in order to improve the efficiency of translation of the polypeptide molecule. A gene encoding a signal peptide of luciferase was introduced in order to secrete the polypeptide molecule expressed by the host cell extracellular. The Spot-Tag-encoding gene was introduced in order to recover and purify polypeptide molecules 2 to 5 from the culture supernatant by affinity purification using an anti-antibody against Spot-Tag VHH.

(2) Transfection of the expression vector

In order to prepare cells producing various polypeptide-molecules, the expression vectors described above were transfected into cells using Expi293 Expression System (Thermofisher Scientific), and the cells were cultured. Specific examples thereof were as follows. The Expi293F(trademark) cell was shaken in an Expi293 Expression Medium at 37° C. in a 5% $CO_2$ (125 rpm). A number of 25.5 mL of cell cultures (3.0 x 10$^6$ cells/mL) was prepared according to the number of samples. An appropriate amount of Opti-MEMI (trademark) was added to each expression vector (about 25 $\mu$g) to make 1.5 mL, and the mixture was gently stirred to prepare a DNA solution. ExpiFectamine (trademark) 293 reagent (80 $\mu$L) and Opti-MEMI (1.4 mL) were gently stirred and allowed to stand at room temperature for 5 minutes to prepare a transfection reagent. The DNA solution and the transfection reagent were gently stirred and allowed to stand at room temperature for 15 minutes. The obtained mixed solution (3 mL) was added to the cell culture (25 mL) and shaken at 37° C. for 20 hours under a 5% $CO_2$ (125 rpm). After 20 hours, 150 $\mu$L and 1.5 mL of ExpiFectamine transfection enhancer 1 and 2 were added to the cultures, and the cultures were shaken at 37°C for 5 days (125 rpm) under a 5% $CO_2$. The culture supernatant contained each polypeptide molecule secreted from the transfected Expi293F cell.

The configurations of the polypeptide molecules were as shown in Fig. 9B. In Fig. 9B, "Luc-signal" represents a signal peptide of luciferase (SEQ ID NO: 14), "CD9(ECD)" represents an ECD of CD9 (SEQ ID NO: 3), "Linker" represents GS1 peptide linker (SEQ ID NO: 8), "BIAP II" represents BIAP II (SEQ ID NO: 1) which is bovine small intestine-derived ALP, "Tag" represents Spot-Tag (SEQ ID NO: 15), "Annexin" represents an epitope region of Annexin A1 (SEQ ID NO: 7), and "TNAP" illustrates full-length human tissue non-specific ALP (SEQ ID NO: 2). The polypeptide molecules 1 to 8 were fusion proteins each containing the various proteins shown in Fig. 9B in order from the N-terminus as a series of polypeptide chains.

(3) Recovery and purification of polypeptide molecule

(3.1) Polypeptide molecules 1 and 6 to 8

Each of the polypeptide molecules described above was recovered and purified by size exclusion column chromatography (SEC). Specific examples thereof were as follows. First, the culture supernatant was recovered 5 days after the transfection. The recovered culture supernatant was transferred to a centrifuge tube, centrifuged (3000 $\times$ g, 30 minutes, 4°C), and the supernatant was then transferred to a new centrifuge tube. 5 mL of the supernatant was concentrated to 0.5 mL using Amicon Ultra-4, 30k (Merck). The concentrated supernatant (0.5 mL) was purified by gel filtration using AKTA pure25 (GE Healthcare) and Superdex 200 Increase10 / 300 GL (GE Healthcare). In the gel filtration, the injection volume was set to 500 $\mu$L, and the running buffer was separated with use of ALP buffer (100 mM triethanolamine (TEA), 150 mM NaCl, 1 mM $MgCl_2$, 0.1 mM $ZnCl_2$, pH 7.0) at a flow rate of 0.6 mL/min.

(3.2) Polypeptide molecules 2 to 5

The polypeptide-molecules described above were recovered and purified from the culture supernatant by affinity purification using an anti-antibody Spot-Tag VHH. Specific examples thereof were as follows.

(i) Concentration of the culture supernatant

The culture supernatant was recovered 5 days after the transfection. The recovered culture supernatant was transferred to a centrifuge tube, centrifuged (3000 g, 30 minutes, 4° C.), and the supernatant was then transferred to a new centrifuge tube. 5 mL of the supernatant was concentrated to 1 mL using Amicon Ultra-4, 30k (Merck).

(ii) Equilibration of Spot-Cap (registered trademark)

0.1 mL (50% slurry) of Spot-Cap (Proteintech) was dispensed into a 1.5 mL low-adsorption tube, centrifuged (2500$\times$g, 2 minutes), and the supernatant was then pipetted off. 0.5 mL of liquid medium was added to the tube, the plate was inverted and mixed to equilibrate Spot-Cap. The tube was centrifuged (2500 $\times$ g, 2 minutes), and the supernatant was then removed by pipetting. The equilibration with the liquid medium was repeated three times in total. The Spot-Cap was agarose beads on which anti-antibody against Spot-Tag VHH was immobilized. The above broth was prepared by mixing 2.5 mL of Expi293 Expression Medium and 0.29 mL of Opti-MEMI.

(iii) Purification by Spot-Cap

The concentrated culture supernatant (1 mL) was filtered through a 0.22 $\mu$m filter, added to a tube containing equilibrated Spot-Cap, and mixed with a rocking shaker at 4°C for 1 hour. The tube was centrifuged (2500 $\times$ g, 2 minutes), and the supernatant was then removed by pipetting. To the tube was added 1 mL of ice-cooled 50 mM acetic acid/sodium acetate buffer (pH 4.5) to wash the Spot-Cap. The tube was centrifuged (2500 $\times$ g, 2 minutes), and the supernatant was then removed by pipetting. The washing with buffer was repeated three times in total. In the third washing, Spot-Cap was transferred to a new 1.5-mL low-adsorption tube, the supernatant was removed, 1 mL of ice-cold ALP buffer was added to the tube, the mixture was inverted and mixed, and the pH was returned to near neutrality. The tube was centrifuged (2500 $\times$

g, 2 minutes), and the supernatant was then removed by pipetting. 100 μL of ice-cooled elution buffer (ALP buffer containing 100 μM of Spot peptide) was added to the tube, and the mixture was mixed using a mixer at 4°C for 5 to 10 minutes. Contact with an elution buffer containing the Spot peptide competitively eluted the polypeptide molecule captured on the Spot-Cap. The tube was centrifuged (2500 × g, 2 minutes), and the supernatant was then collected in a new 1.5 mL low-adsorption tube. The elution and recovery with the elution buffer were repeated 5 or 6 times in total.

Example 1: Measurement using a calibrator containing polypeptide molecule 1

In Example 1, a calibrator containing the polypeptide molecule 1 was prepared from the fractions recovered and purified by SEC, and used for measurement of protein concentration and ALP activity of EV. The polypeptide molecule 1 was, as shown in Fig. 9B, a fusion protein that contains, in order from the N-terminus, a signal peptide of luciferase, CD9(ECD), GS1 peptide linker, and BIAP II as a series of polypeptide chains.

(1) Preparation of calibrator

For each fraction acquired by SEC, the total ALP activity of the fractions was measured by a fully automatic immunoassay device HI-1000 (Sysmex Corporation). Fig. 10 illustrates results of the SEC analysis (upper chromatogram) and measured results of the ALP activity of each fraction (lower graph). Referring to Fig. 10, as shown in the area surrounded by squares, the fraction containing the peak in the center of the chromatogram demonstrated high ALP activity. These fractions were fractions B2, B3 and B4. It was considered that the fractions B2, B3, and B4 contained polypeptide molecule 1. Equal amounts of fractions B2, B3 and B4 were mixed, and the obtained mixture was analyzed by SDS-PAGE under reducing conditions. The results of CBB staining of the gel after electrophoresis are shown in Fig. 11. Referring to Fig. 11, it was shown that the polypeptide molecule 1 is contained in a mixture of fractions B2, B3, and B4. A mixture of fractions B2, B3 and B4 was used as a calibrator of Example 1 in the subsequent experiment.

(2) Measurement of ALP activity of calibrator

(2.1) Preparation of measurement reagent (R1 reagent) containing capture antibody

As the capture antibody, a commercially available anti-CD9 antibody (Clone:H19a) was used. The capture antibody was reduced with 0.3 M 2-mercaptoethylamine (MEA) solution, and gel-filtered with Amicon Ultra-4, 30k (Merck) to obtain reduced antibody. The reduced antibody was mixed with biotin-PEAC$_5$ maleimide (Dojin Chemical Laboratory Corporation) and reacted at 4° C. overnight. The resulting solution was gel-filtered through Amicon Ultra-4, 30k (Merck), to obtain a biotin-labeled capture antibody. The biotin-labeled capture antibody was added to an R1 buffer having the following composition so as to have a concentration of 1 μg/mL to prepare an R1 reagent. As a control, a reagent in which control IgG was added to the R1 buffer instead of the capture antibody was prepared.

<u>Composition of R1 buffer</u>

| | |
|---|---|
| MES (pH6.5) | 0.1 M |
| NaCl | 300 mM |
| MgCl$_2$, 6H$_2$O | 10 mM |
| ZnCl$_2$ | 0.1 mM |
| BSA | 1% |
| Casein Na | 0.5% |

(2.2) Another reagent

As a measurement reagent (R2 reagent) containing a solid phase, a HISCL R2 reagent (Sysmex Corporation) containing streptavidin-coupled magnetic particles was used. As the measurement buffer (R4 reagent), a MES buffer (10.9 mM MES, pH6.5 and 3 mM MgCl$_2$ 6H$_2$O) was used. As a substrate solution of ALP (R5 reagent), a HISCL R5 reagent (Sysmex Corporation) containing CDP-star (trademark) was used.

(2.3) Measurement of ALP activity

The ALP activity of the calibrator was measured by HI-1000 using the above-described respective reagents. The measurement by HI-1000 was based on sandwich ELISA, wherein a polypeptide molecule in the calibrator was immobilized on a magnetic particle by a capture antibody, and ALP activity of the polypeptide molecule was measured. The measurement conditions of HI-1000 are shown in Table 1. Specific operations were as follows. After adding the R1 reagent (110 μL) to the cuvette, a calibrator (20 μL) was added and mixed, and the mixture was allowed to stand for about 166 seconds. The R2 reagent (30 μL) was added to the cuvette and mixed, and the mixture was allowed to stand for about 382 seconds. The magnetic particle in the mixed solution was magnetically collected to remove the supernatant, and a HISCL washing solution (300 μL) was added to wash the magnetic particle. The supernatant was removed, and the R4 reagent (50 μL) and the R5 reagent (100 μL) were added to the cuvette and mixed, and allowed to stand for about 305 seconds. Then, the emission intensity (count) was acquired as a measured value of the signal. The measurement was repeated three times, and average values of the three measured values were acquired. Measurement using a reagent containing control IgG instead of the R1 reagent was also performed in the same manner.

[Table 1]

| REAGENT/SAMPLE | DISPENSING VOLUME (μL) | REACTION TIME (SECONDS) |
|---|---|---|
| R1 | 110 | - |
| CALIBRATOR | 20 | 166 |
| R2 | 30 | 382 |
| R4 | 50 | - |
| R5 | 100 | 305 |

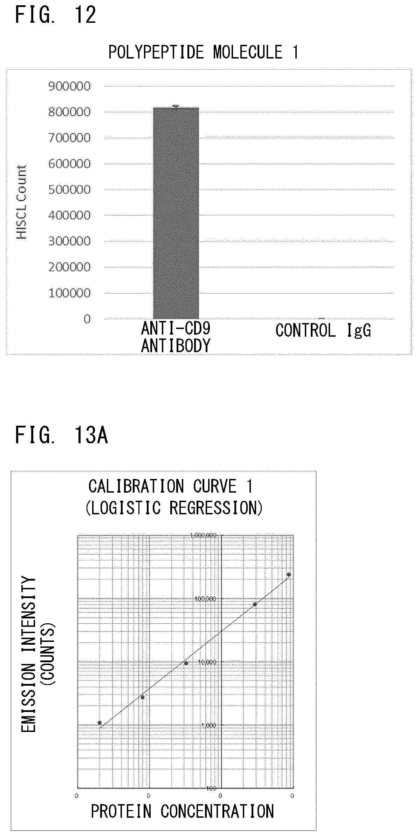

The results are shown in Fig. 12. As shown in Fig. 12, significantly high signal measured values were acquired when the calibrator was measured using an R1 reagent containing a biotin-labeled anti-CD9 antibody. However, when a reagent containing control IgG was used instead of the R1 reagent, little signal was detected. In this way, when an anti-CD9 antibody capable of specifically capturing the polypeptide molecule 1 was used, the activity of ALP contained in the polypeptide molecule 1 could be measured. Therefore, it was shown that the polypeptide molecule 1 can be used for measurement of ALP activity by immunoassay.

(3) Preparation of a set of calibrators

(3.1) Valuing of protein concentration and ALP activity of calibrator

A mixture of the aforementioned fractions B2, B3, and B4 was used as a stock solution of a calibrator. The protein concentration of this stock solution was measured on the basis of absorbance at 280 nm using a spectrophotometer NanoDrop™-1000 (Thermofisher Scientific). The protein concentration of the stock solution was found to be 0.204 mg/mL. The stock solution was diluted 100-fold with a calibrator dilution buffer (1M Tris-HCl (pH7.5), 150 mM NaCl, 10 mM MgCl$_2$ 6H$_2$O, 1% BSA). For this diluted solution, the ALP activity value was biochemically measured based on an IFCC standard measurement operation method. This measurement was performed by Hitachi automatic analyzer 7180 (Hitachi High-Technologies Corporation) using Iatro (registered trademark) ALP-IF R1/R2 (LSI Medience Corporation) as a measurement reagent and enzyme calibrator plus (trademark) (Sysmex Corporation) as a standard substance. The ALP activity value of the diluted solution was found to be 31.4233 U/L. Therefore, the ALP activity value of the stock solution was determined to be 3142.33

U/L.

(3.2) Preparation of calibrators C0 to C5

Five calibrators C1 to C5 having different protein concentrations were prepared by diluting the stock solution of the calibrator with the calibrator dilution buffer based on the weight method. A calibrator dilution buffer was used as the calibrator C0. The protein concentrations and ALP activity values of the six calibrators C0 to C5 are shown in Table 2.

[Table 2]

| CALIBRATOR | C0 | C1 | C2 | C3 | C4 | C5 |
|---|---|---|---|---|---|---|
| PROTEIN CONCENTRATION (μg/mL) | 0 | 0.0002024 | 0.0008096 | 0.0032386 | 0.029147 | 0.0874411 |
| ALP ACTIVITY VALUE (U/L) | 0 | 0.0031178 | 0.0124714 | 0.0498854 | 0.4489689 | 1.3469067 |

(4) Measurement of EV in a sample using a calibrator

(4.1) Preparation of calibration curve of protein concentration

Using the above-described R1, R2, R4, and R5 reagents, the ALP activity of the calibrators C0 to C5 was measured by HI-1000, and emission intensity (count value) was acquired. The measurement was performed three times for each calibrator. The results are shown in Table 3. "AV", "SD", and "CV" in the table are the average value, standard deviation, and coefficient of variation of the emission intensity obtained by three measurements, respectively. A calibration curve was prepared on the basis of logistic regression, using the protein concentrations of the calibrators shown in Table 2 and the average values of the emission intensities of the calibrators shown in Table 3. This calibration curve is referred to as "calibration curve 1" and is shown in Fig. 13A.

[Table 3]

| | C0 | C1 | C2 | C3 | C4 | C5 |
|---|---|---|---|---|---|---|
| | 615 | 1057 | 2742 | 9454 | 79020 | 234756 |
| | 593 | 1139 | 2701 | 9401 | 83719 | 243299 |
| | 573 | 1042 | 2724 | 9629 | 79736 | 239335 |
| AV | 593.66667 | 1079.3333 | 2722.3333 | 9494.6667 | 80825 | 239130 |
| SD | 17.152907 | 42.632799 | 16.779617 | 97.421193 | 2067.1383 | 3490.6762 |
| CV | 3% | 4% | 1% | 1% | 3% | 1% |

The emission intensity of each calibrator shown in Table 3 was applied to calibration curve 1 to obtain the protein concentration (μg/mL) as an actual measured value. For each calibrator, the protein concentration obtained from the emission intensity and the protein concentration (referred to as theoretical concentration) shown in Table 2 are shown in Table 4 below. As can be seen from Table 4, for the calibrators C1 to C5, the protein concentration obtained from the emission intensity using a calibration curve was found to be a value very close to the theoretical concentration. This showed that the protein concentration converted from the signal measured value acquired by the immunoassay using the capture antibody was almost the same as the protein concentration acquired by the absorbance measurement. Therefore, it was shown that the polypeptide molecule 1 can be used as a standard substance for quantifying the protein concentration by immunoassay using a capture antibody.

[Table 4]

| | C0 | C1 | C2 | C3 | C4 | C5 |
|---|---|---|---|---|---|---|
| THEORETICAL CONCENTRATION (μg/mL) | 0 | 0.0002024 | 0.0008096 | 0.0032386 | 0.029147 | 0.0874411 |
| | 9.709E-06 | 0.0001895 | 0.0008336 | 0.0032828 | 0.0278206 | 0.0863292 |
| | 0 | 0.0002218 | 0.0008182 | 0.0032638 | 0.0294934 | 0.0897598 |
| | 0 | 0.0001836 | 0.0008269 | 0.0033457 | 0.0280752 | 0.0881647 |
| AV | 3.236E-06 | 0.0001983 | 0.0008263 | 0.0032974 | 0.0284631 | 0.0880846 |
| SD | 4.577E-06 | 1.679E-05 | 6.293E-06 | 3.498E-05 | 0.0007359 | 0.0014017 |
| CV | 141% | 8% | 1% | 1% | 3% | 2% |

(4.2) Preparation of calibration curve of ALP activity value

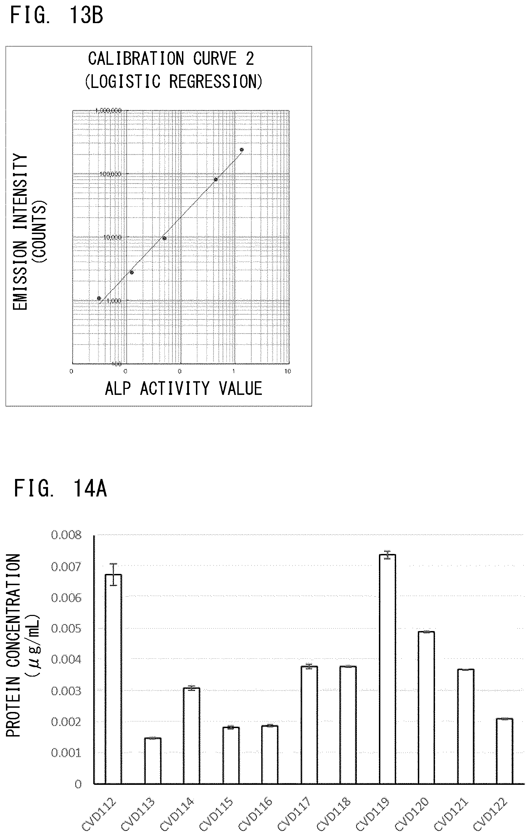

A calibration curve was prepared based on logistic regression using the ALP activity values of the calibrators shown in Table 2 and the average values of the emission intensities of the calibrators shown in Table 3. This calibration curve is referred to as "calibration curve 2", and is shown in Fig. 13B. The emission intensity of each calibrator shown in Table 3 was applied to calibration curve 2, and an ALP activity value (U/L) as an actual measured value was acquired. For each calibrator, the ALP activity value obtained from the emission intensity and the ALP activity value (referred to as theoretical ALP activity) shown in Table 2 are shown in Table 5 below. As can be seen from Table 5, for the calibrators C1 to C5, the ALP activity value obtained from the emission intensity using a calibration curve was a value very close to the theoretical ALP activity. This showed that the ALP activity value converted from the signal measured value acquired by the immunoassay using the capture antibody was almost the same as the ALP activity value acquired by the biochemical measurement. Therefore, it was shown that the polypeptide molecule 1 can be used as a standard substance for quantifying ALP activity by immunoassay using a capture antibody.

[Table 5]

| | C0 | C1 | C2 | C3 | C4 | C5 |
|---|---|---|---|---|---|---|
| THEORETICAL ALP ACTIVITY (U/L) | 0 | 0.0031178 | 0.0124714 | 0.0498854 | 0.4489689 | 1.3469067 |
| | 0.0001496 | 0.0029189 | 0.0128408 | 0.0505673 | 0.4285373 | 1.3297804 |
| | 0 | 0.0034162 | 0.012604 | 0.0502741 | 0.4543044 | 1.3826238 |
| | 0 | 0.0028276 | 0.0127369 | 0.0515351 | 0.4324593 | 1.3580537 |
| AV | 4.985E-05 | 0.0030543 | 0.0127272 | 0.0507922 | 0.4384337 | 1.3568193 |
| SD | 7.05E-05 | 0.0002586 | 9.693E-05 | 0.0005388 | 0.011336 | 0.0215909 |
| CV | 141% | 8% | 1% | 1% | 3% | 2% |

(4.3) Measurement of protein concentration of EV and ALP activity in a sample

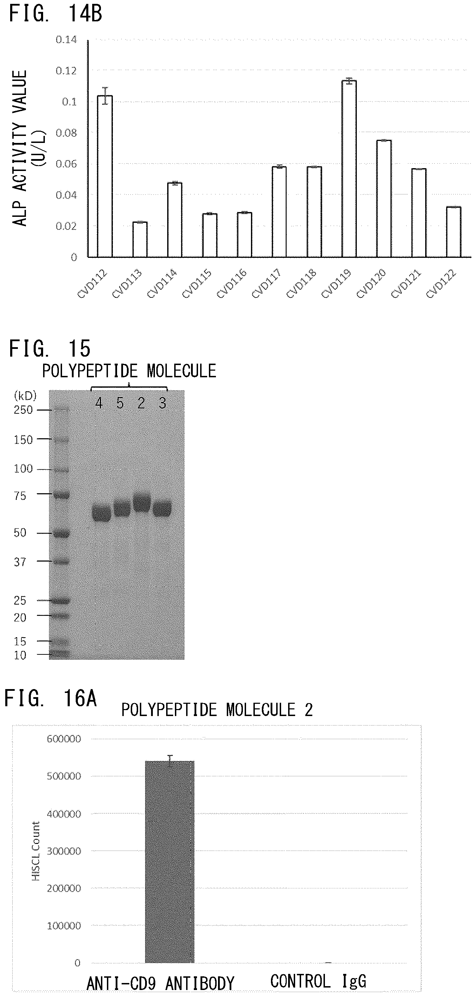

The frozen plasma of 11 patients with a history of cardiovascular disease (CVD) was thawed, and ALP activity of each sample was measured by HI-1000 using the R1, R2, R4, and R5 reagents described above, to obtain emission intensity (count value). The measurement was performed three times for each sample. The emission intensity of each sample was applied to calibration curve 1 to obtain the protein concentration of EV in the sample ($\mu$g/mL). The results are illustrated in Fig. 14A. The emission intensity of each sample was applied to calibration curve 2 to obtain an activity value (U/L) of ALP contained in EV in the sample. The results are shown in Fig. 14B. In Figs. 14A and B, the error bars represent $\pm$1SD. As shown in these figures, the protein concentration and the ALP activity value of EV were acquired from the signal measured values of the samples containing EV by using a calibration curve prepared from the signal measured values of the calibrator containing the polypeptide molecule 1.

(5) Evaluation of calibrator quality

(5.1) Storage stability

The calibrators C0 to C5 were cryopreserved at -80° C. for 30 days, and then thawed. The ALP activity of each thawed calibrator was measured by HI-1000 using the above-described R1, R2, R4, and R5 reagents, and emission intensity (count value) was acquired. The measurement was performed three times for each calibrator. In the same manner as in (4.1), a calibration curve of the protein concentration was prepared using the protein concentration of each calibrator and the average value of the emission intensities of each calibrator. The emission intensity of each calibrator was applied to this calibration curve to obtain the protein concentration ($\mu$g/mL). In the table, "Day 0" indicates the protein concentration at which the calibration curve could be prepared by measuring each calibrator in the same manner immediately before cryopreservation. The percentage in the third row of the table illustrates the ratio of the protein concentration on Day 30 to the protein concentration on Day 0 of each calibrator. As can be seen from Table 6, no significant change in protein concentration was observed between Day 0 and Day 30. Therefore, it was found that the calibrator containing the polypeptide molecule 1 can be stably stored by freezing.

[Table 6]

|  | C0 | C1 | C2 | C3 | C4 | C5 |  |
|---|---|---|---|---|---|---|---|
| Day0 | 0.0000000 | 0.0001983 | 0.0008263 | 0.0032974 | 0.0284627 | 0.0880824 | μg/mL |
| Day30 | 0.0000000 | 0.0001934 | 0.0008551 | 0.0033062 | 0.0280052 | 0.0884681 | μg/mL |
|  |  | 98% | 103% | 100% | 98% | 100% |  |

(5.2) On-board stability

The calibrators C0 to C5 were set in HI-1000 and allowed to stand at room temperature for 6 hours. Thereafter, the ALP activity of each calibrator was measured by HI-1000 using the above-described R1, R2, R4, and R5 reagents, and emission intensity (count value) was acquired. The measurement was performed three times for each calibrator. In the same manner as in the aforementioned (4.1), a calibration curve of protein concentration was prepared using the protein concentration of each calibrator and an average value of the emission intensities of each calibrator, and the emission intensity of each calibrator was applied to this calibration curve to obtain protein concentration (μg/mL). Average protein concentrations of each calibrator are shown in Table 7. In the table, "0h" indicates the protein concentration at which the calibration curve could be prepared by measuring each calibrator in the same manner immediately after the setting to HI-1000. The percentage in the third row of the table illustrates the ratio of the protein concentration of 6h to the protein concentration of 0h for each calibrator except for C0. As can be seen from Table 7, no significant change in protein concentration was observed between 0h and 6h. Therefore, the calibrator containing the polypeptide molecule 1 was found to demonstrate good on-board stability.

[Table 7]

|  | C0 | C1 | C2 | C3 | C4 | C5 |  |
|---|---|---|---|---|---|---|---|
| 0h | 0 | 0.000193439 | 0.0008551 | 0.0033062 | 0.0280052 | 0.0884681 | μg/mL |
| 6h | 0 | 0.000196943 | 0.0008336 | 0.0033028 | 0.0283031 | 0.0882265 | μg/mL |
|  |  | 102% | 97% | 100% | 101% | 100% |  |

(5.3) Simultaneous repeatability

In the same manner as in the aforementioned (4.1), a calibration curve of the protein concentration was prepared using the protein concentrations of each calibrator C0 to C5 and the average value of the emission intensities of each calibrator. The stock solution of the calibrator containing the polypeptide molecule 1 was diluted by the weight method with a calibrator dilution buffer to prepare three calibrators L, M and H having different protein concentrations. The ALP activity of each calibrator was measured by HI-1000 using the above-described R1, R2, R4, and R5 reagents, and emission intensity (count value) was acquired. The measurement was repeated 10 times for each calibrator. The emission intensity of each calibrator was applied to a calibration curve to obtain a protein concentration (μg/mL). For each calibrator, the protein concentrations obtained for each measurement (AV), standard deviation (SD), and coefficient of variation (CV) are shown in Table 8. In the table, the first to tenth values of each calibrator and the value of AV are protein concentrations (μg/mL).

[Table 8]

|  | L | M | H |
|---|---|---|---|
| FIRST | 0.000272 | 0.001079 | 0.006343 |
| SECOND | 0.000276 | 0.001068 | 0.00624 |
| THIRD | 0.000266 | 0.001071 | 0.006252 |
| 4TH | 0.000285 | 0.001031 | 0.006366 |
| 5TH | 0.000266 | 0.001049 | 0.006493 |
| 6TH | 0.000287 | 0.001053 | 0.006129 |
| 7TH | 0.000284 | 0.00105 | 0.006132 |
| 8TH | 0.000262 | 0.00101 | 0.006172 |
| 9TH | 0.000244 | 0.00105 | 0.006123 |
| 10TH | 0.000259 | 0.001019 | 0.006195 |
| AV | 0.00027 | 0.001048 | 0.006244 |
| SD | 1.29E-05 | 2.1E-05 | 0.000116 |
| CV | 4.8% | 2.0% | 1.9% |

As shown in Table 8, the CV values of all calibrators were 5% or less. Therefore, it was found that the calibrator containing the polypeptide molecule 1 was excellent in simultaneous repeatability.

(5.4) Inter-day reproducibility

The ALP activity of each calibrator C0 to C5 was measured on Day 1 by HI-1000 using the R1, R2, R4, and R5 reagents described above, and emission intensity (count value) was acquired. The measurement was performed three times for each calibrator. In the same manner as in the aforementioned (4.1), a calibration curve of the protein concentration was prepared using the protein concentration of each calibrator and the average value of the emission intensities of each calibrator. The ALP activity of each of calibrators L, M, and H was measured three times daily, and the measurement was repeated for 5 days. The measurement was performed by HI-1000 using the above-described R1, R2, R4, and R5 reagents, and emission intensity (count value) was acquired. The protein concentration ($\mu$g/mL) was acquired by applying the emission intensity of each calibrator to a calibration curve prepared on Day 1. On each measurement day, a deviation (%) between the concentration (measured value) obtained from the emission intensity and the protein concentration (theoretical value) obtained by absorbance measurement was calculated by the following formula. The results are shown in Table 9.

Deviation (%)=[1-(theoretical value-measured value)/theoretical value]x100

[Table 9]

|  | L | M | H |
|---|---|---|---|
| Day1 | 98% | 96% | 98% |
| Day2 | 101% | 99% | 97% |

(continued)

|      | L    | M   | H   |
|------|------|-----|-----|
| Day3 | 97%  | 98% | 99% |
| Day4 | 102% | 98% | 97% |
| Day5 | 101% | 97% | 98% |

As shown in Table 9, the deviation values of all calibrators were 100 ± 5%. Therefore, the calibrator containing the polypeptide molecule 1 was found to be excellent in inter-day repeatability.

Example 2: Measurement using a calibrator containing polypeptide molecules 2 to 5

In Example 2, a calibrator containing each of the polypeptide molecules 2 to 5 was prepared from each eluent acquired by affinity purification, and the protein concentration and ALP activity of each calibrator were measured. The configurations of the polypeptide molecules 2 to 5 were as shown in Fig. 9B. The polypeptide molecule 2 was a fusion protein further including a GS1 peptide linker and Spot-Tag at the C-terminus of BIAP II in the polypeptide molecule 1. The polypeptide molecule 3 was a fusion protein in which the position of CD9(ECD) and BIAP II was changed in the polypeptide molecule 2. The polypeptide molecule 4 was a fusion protein in which CD9(ECD) was replaced with an epitope region of Annexin A1 in the polypeptide molecule 3. The polypeptide molecule 5 was a fusion protein further including an epitope region of Annexin A1 and a GS1 peptide linker between the signal peptide of luciferase and BIAP II in the polypeptide molecule 4.

(1) Preparation of calibrator

Each eluent acquired by affinity purification using Spot-Cap was subjected to SDS-PAGE analysis under reducing conditions. The result of CBB staining of the gel after electrophoresis is shown in Fig. 15. With reference to Fig. 15, it was shown that the polypeptide molecules 2 to 5 could be acquired. An eluent containing each of the polypeptide molecules 2 to 5 was used as a calibrator of Example 2 in the subsequent experiment.

(2) Measurement of ALP activity of calibrator

(2.1) Preparation of R1 reagent

For the measurement of the polypeptide molecules 2 and 3, the R1 reagent prepared in Example 1, that is, the R1 reagent containing a biotin-labeled anti-CD9 antibody was used. For measuring the polypeptide molecules 4 and 5, an R1 reagent containing biotin-labeled anti-Annexin A1 antibody (Clone:EH17a) was used as the capture antibody. In preparation of the R1 reagent, first, reduction and biotin labeling were performed on an anti-Annexin A1 antibody in the same manner as in Example 1 to obtain a biotin-labeled anti-Annexin A1 antibody. Then, the obtained labeled antibody is added to the R1 buffer so as to have a concentration of 1 μ g / mL, and biotin-labeled anti-Annexin A1 is anti-bound An R1 reagent containing the body was prepared. As a control, a reagent in which control IgG was added to the R1 buffer was used. The same R2, R4 and R5 reagents as in Example 1 were used.

(2.2) Measurement of ALP activity

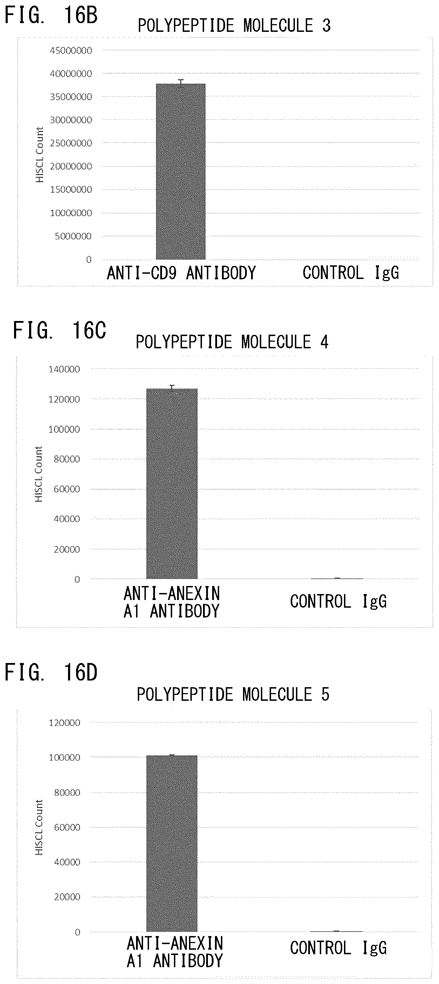

The ALP activity of each calibrator was measured by HI-1000 in the same manner as in Example 1 using the above-described R1, R2, R4, and R5 reagents. The results are shown in Figs. 16A to D. As shown in these figures, remarkably high signal measured values were acquired when the calibrator was measured using the R1 reagent containing the capture antibody. However, when a reagent containing control IgG was used instead of the R1 reagent, little signal was detected. In this way, when a capture antibody capable of specifically capturing each polypeptide molecule was used, the activity of ALP contained in the polypeptide molecule could be measured. Therefore, it was shown that all of the polypeptide molecules 2 to 5 can be used for measurement of ALP activity by immunoassay.

(3) Preparation of a set of calibrators containing polypeptide molecule 2

(3.1) Valuing of protein concentration and ALP activity of calibrator

The eluent obtained by affinity purification was used as a stock solution of the calibrator. The protein concentration of this stock solution was measured in the same manner as Example 1 by NanoDrop-1000. The protein concentration of the stock solution was found to be 0.600 mg/mL. The stock solution was diluted 70-fold with the calibrator dilution buffer, and the ALP activity of the diluted solution was biochemically measured in the same manner as in Example 1. The ALP activity value of the diluted solution was found to be 6.555 U/L. Therefore, the ALP activity value of the stock solution was determined to be 458.87 U/L.

(3.2) Preparation of calibrators C0 to C5

Five calibrators C1 to C5 having different protein concentrations were prepared by diluting a stock solution of the calibrator containing the polypeptide molecule 2 by a weight method based on a calibrator dilution buffer. A calibrator dilution buffer was used as the calibrator C0. The protein concentrations and ALP activity values of the six calibrators C0 to C5 are shown in Table 10.

[Table 10]

| CALIBRATOR | C0 | C1 | C2 | C3 | C4 | C5 |
|---|---|---|---|---|---|---|
| PROTEIN CONCENTRATION ($\mu$g/mL) | 0 | 0.0190523 | 0.0781591 | 0.3126365 | 2.8137288 | 8.5406859 |
| ALP ACTIVITY VALUE (U/L) | 0 | 0.0146233 | 0.0592880 | 0.2400679 | 2.1596384 | 6.5552846 |

(3.3) Preparation of calibration curve of protein concentration

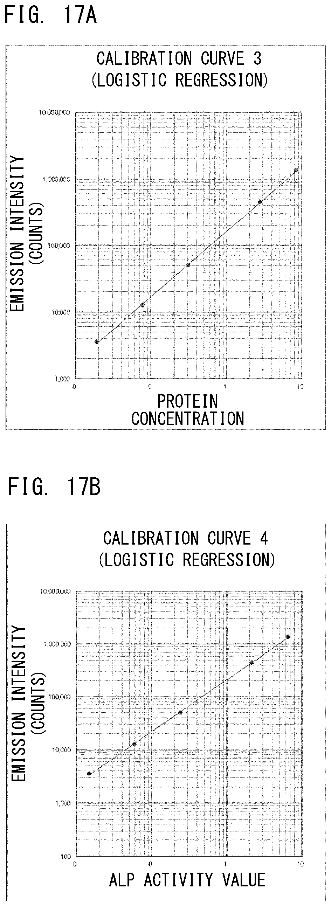

Using the above-described R1, R2, R4, and R5 reagents, the ALP activity of the calibrators C0 to C5 was measured by HI-1000, and emission intensity (count value) was acquired. The measurement was performed three times for each calibrator. The results are shown in Table 11. "AV", "SD" and "CV" in the table are the same as Table 3 in Example 1. A calibration curve was prepared on the basis of logistic regression, using the protein concentrations of the calibrators shown in Table 10 and the average values of the emission intensities of the calibrators shown in Table 11. This calibration curve is referred to as "calibration curve 3", and is shown in Fig. 17A.

[Table 11]

| | C0 | C1 | C2 | C3 | C4 | C5 |
|---|---|---|---|---|---|---|
| | 469 | 3608 | 12647 | 50846 | 442320 | 1366337 |
| | 430 | 3428 | 12507 | 50531 | 451171 | 1359817 |
| | 430 | 3635 | 12849 | 49753 | 450098 | 1395476 |
| AV | 443 | 3557 | 12667.667 | 50376.667 | 447863 | 1373876.7 |
| SD | 18.384776 | 91.880357 | 140.3836 | 459.36647 | 3943.8956 | 15503.247 |
| CV | 4% | 3% | 1% | 1% | 1% | 1% |

The emission intensity of each calibrator shown in Table 11 was applied to calibration curve 3 to obtain the protein concentration ($\mu$g/mL) as an actual measured value. For each calibrator, the protein concentration obtained from the emission intensity and the protein concentration (theoretical concentration) shown in Table 10 are shown in Table 12 below. As can be seen from Table 12, for the calibrators C1 to C5, the protein concentration obtained from the emission intensity using a calibration curve was found to be a value very close to the theoretical concentration. Therefore, it was shown that the polypeptide molecule 2 can be used as a standard substance for quantifying the protein concentration by immunoassay using a capture antibody, similarly to the polypeptide molecule 1.

[Table 12]

|  | C0 | C1 | C2 | C3 | C4 | C5 |
|---|---|---|---|---|---|---|
| THEORETICAL CONCENTRATION ($\mu$g/mL) | 0 | 0.0190523 | 0.0772446 | 0.3127773 | 2.8137288 | 8.5406859 |
|  | 0.0001603 | 0.019656 | 0.075948 | 0.3143549 | 2.7660504 | 8.5725572 |
|  | 0 | 0.0185365 | 0.0750755 | 0.3123873 | 2.8215649 | 8.5315205 |
|  | 0 | 0.019824 | 0.077207 | 0.3075275 | 2.8148347 | 8.7559674 |
| AV | 5.343E-05 | 0.0193388 | 0.0760768 | 0.3114233 | 2.8008167 | 8.6200151 |
| SD | 7.557E-05 | 0.0005715 | 0.000875 | 0.0028694 | 0.0247365 | 0.0975817 |
| CV | 141% | 3% | 1% | 1% | 1% | 1% |

(3.4) Preparation of calibration curve of ALP activity value

A calibration curve was prepared based on logistic regression using the ALP activity values of the calibrators shown in Table 10 and the average values of the emission intensities of the calibrators shown in Table 11. This calibration curve is referred to as "calibration curve 4", and is shown in Fig. 17B. The emission intensity of each calibrator shown in Table 11 was applied to a calibration curve 4, and an ALP activity value (U/L) as an actual measured value was acquired. For each calibrator, the ALP activity value obtained from the emission intensity and the ALP activity value (theoretical ALP activity) shown in Table 10 are shown in Table 13 below. As can be seen from Table 13, for the calibrators C1 to C5, the ALP activity value obtained from the emission intensity using a calibration curve was a value very close to the theoretical ALP activity. Therefore, it was shown that the polypeptide molecule 2 can be used as a standard substance for quantifying ALP activity by immunoassay using a capture antibody, similarly to the polypeptide molecule 1.

[Table 13]

|  | C0 | C1 | C2 | C3 | C4 | C5 |
|---|---|---|---|---|---|---|
| THEORETICAL ALP ACTIVITY (U/L) | 0 | 0.0146233 | 0.059288 | 0.2400679 | 2.1596384 | 6.5552846 |
|  | 0.000123 | 0.0150867 | 0.0582928 | 0.2412788 | 2.1230435 | 6.5797469 |
|  | 0 | 0.0142274 | 0.0576231 | 0.2397685 | 2.1656528 | 6.5482498 |
|  | 0 | 0.0152156 | 0.0592592 | 0.2360385 | 2.1604872 | 6.7205209 |
| AV | 4.101E-05 | 0.0148433 | 0.0583917 | 0.2390286 | 2.1497278 | 6.6161725 |
| SD | 5.8E-05 | 0.0004386 | 0.0006716 | 0.0022024 | 0.0189862 | 0.0748975 |
| CV | 141% | 3% | 1% | 1% | 1% | 1% |

(4) Preparation of a set of calibrators containing polypeptide molecule 3

(4.1) Valuing of protein concentration and ALP activity of calibrator

The eluent obtained by affinity purification was used as a stock solution of the calibrator. The protein concentration of this stock solution was measured in the same manner as Example 1 by NanoDrop-1000. The protein concentration of the stock solution was found to be 0.572 mg/mL. The stock solution was diluted 55000-fold with the calibrator dilution buffer, and the ALP activity of the diluted solution was biochemically measured in the same manner as in Example 1. The ALP activity value of the diluted solution was found to be 2.07182 U/L. Therefore, the ALP activity value of the stock solution was determined to be 113950 U/L.

(4.2) Preparation of calibrators C0 to C5

Five calibrators C1 to C5 having different protein concentrations were prepared by diluting the stock solution of the calibrator containing the polypeptide molecule 3 by the weight method based on the calibrator dilution buffer. A calibrator dilution buffer was used as the calibrator C0. The protein concentrations and ALP activity values of the six calibrators C0 to C5 are shown in Table 14.

[Table 14]

| CALIBRATOR | C0 | C1 | C2 | C3 | C4 | C5 |
|---|---|---|---|---|---|---|
| PROTEIN CONCENTRATION ($\mu$g/mL) | 0 | 0.0013228 | 0.0052910 | 0.0211640 | 0.1904762 | 0.5714286 |
| ALP ACTIVITY VALUE (U/L) | 0 | 0.0047959 | 0.0191835 | 0.0767341 | 0.6906071 | 2.0718213 |

(4.3) Preparation of calibration curve of protein concentration

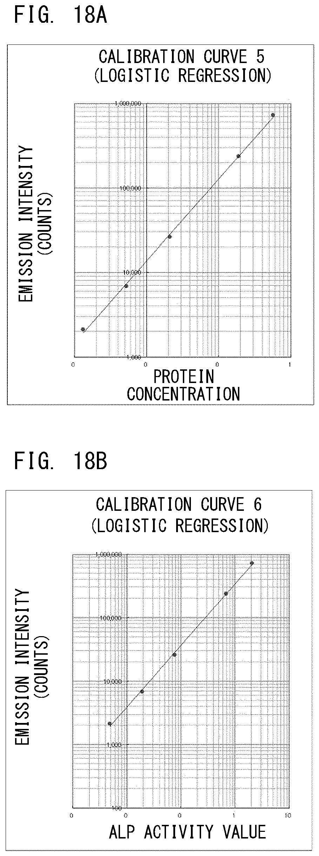

Using the above-described R1, R2, R4, and R5 reagents, the ALP activity of the calibrators C0 to C5 was measured by HI-1000, and emission intensity (count value) was acquired. The measurement was performed three times for each calibrator. The results are shown in Table 15. "AV", "SD" and "CV" in the table are the same as Table 3 in Example 1. A calibration curve was prepared on the basis of logistic regression, using the protein concentrations of the calibrators shown in Table 14 and the average values of the emission intensities of the calibrators shown in Table 15. This calibration curve is referred to as "calibration curve 5", and is shown in Fig. 18A.

[Table 15]

| | C0 | C1 | C2 | C3 | C4 | C5 |
|---|---|---|---|---|---|---|
| | 459 | 2135 | 6862 | 26254 | 235092 | 753041 |
| | 468 | 2089 | 6975 | 26415 | 238681 | 727827 |
| | 443 | 2155 | 6739 | 26093 | 239231 | 704338 |
| AV | 456.66667 | 2126.3333 | 6858.6667 | 26254 | 237668 | 728402 |
| SD | 10.338708 | 27.632509 | 96.375423 | 131.45595 | 1835.2942 | 19887.073 |
| CV | 2% | 1% | 1% | 1% | 1% | 3% |

The emission intensity of each calibrator shown in Table 15 was applied to a calibration curve 5 to obtain a protein concentration ($\mu$g/mL) as an actual measured value. For each calibrator, the protein concentration obtained from the emission intensity and the protein concentration (theoretical concentration) shown in Table 14 are shown in Table 16 below. As can be seen from Table 16, for the calibrators C1 to C5, the protein concentration obtained from the emission intensity using a calibration curve was found to be a value very close to the theoretical concentration. Therefore, it was shown that the polypeptide molecule 3 can be used as a standard substance for quantifying the protein concentration by immunoassay using a capture antibody, similarly to the polypeptide molecule 1.

[Table 16]

| | C0 | C1 | C2 | C3 | C4 | C5 |
|---|---|---|---|---|---|---|
| THEORETICAL CONCENTRATION ($\mu$g/mL) | 0 | 0.0013228 | 0.005291 | 0.021164 | 0.1904762 | 0.5714286 |
| | 1.94E-06 | 0.0013649 | 0.0051859 | 0.0207897 | 0.187738 | 0.6001249 |
| | 9.372E-06 | 0.0013276 | 0.0052771 | 0.020919 | 0.1906006 | 0.5800706 |
| | 0 | 0.0013811 | 0.0050867 | 0.0206604 | 0.1910393 | 0.5613871 |
| AV | 3.771E-06 | 0.0013579 | 0.0051833 | 0.0207897 | 0.1897926 | 0.5805276 |
| SD | 4.039E-06 | 2.24E-05 | 7.777E-05 | 0.0001056 | 0.0014639 | 0.0158179 |
| CV | 107% | 2% | 2% | 1% | 1% | 3% |

(4.4) Preparation of calibration curve of ALP activity value

A calibration curve was prepared based on logistic regression using the ALP activity values of the calibrators shown in Table 14 and the average values of the emission intensities of the calibrators shown in Table 15. This calibration curve is referred to as "calibration curve 6", and is shown in Fig. 18B. The emission intensity of each calibrator shown in Table 15 was applied to a calibration curve 6, and an ALP activity value (U/L) as an actual measured value was acquired. For each calibrator, the ALP activity value obtained from the emission intensity and the ALP activity value (theoretical ALP activity) shown in Table 14 are shown in Table 17 below. As can be seen from Table 17, for the calibrators C1 to C5, the ALP activity value obtained from the emission intensity using a calibration curve was a value very close to the theoretical ALP activity. Therefore, it was shown that the polypeptide molecule 3 can be used as a standard substance for quantifying ALP activity by immunoassay using a capture antibody, similarly to the polypeptide molecule 1.

[Table 17]

| | C0 | C1 | C2 | C3 | C4 | C5 |
|---|---|---|---|---|---|---|
| THEORETICAL ALP ACTIVITY (U/L) | 0 | 0.0047959 | 0.0191835 | 0.0767341 | 0.6906071 | 2.0718213 |
| | 7.033E-06 | 0.0049488 | 0.0188026 | 0.075377 | 0.6806792 | 2.1758653 |
| | 3.398E-05 | 0.0048136 | 0.0191332 | 0.0758458 | 0.6910583 | 2.1031547 |
| | 0 | 0.0050075 | 0.0184428 | 0.0749081 | 0.6926488 | 2.0354142 |
| AV | 1.367E-05 | 0.0049233 | 0.0187929 | 0.075377 | 0.6881288 | 2.1048114 |
| SD | 1.464E-05 | 8.121E-05 | 0.000282 | 0.0003828 | 0.0053075 | 0.0573509 |
| CV | 107% | 2% | 2% | 1% | 1% | 3% |

(5) Preparation of a set of calibrators containing polypeptide molecule 4

(5.1) Valuing of protein concentration and ALP activity of calibrator

The eluent obtained by affinity purification was used as a stock solution of the calibrator. The protein concentration of this stock solution was measured in the same manner as Example 1 by NanoDrop-1000. The protein concentration of the stock solution was found to be 0.682 mg/mL. The stock solution was diluted 1000-fold with the calibrator dilution buffer, and the ALP activity of the diluted solution was biochemically measured in the same manner as in Example 1. The ALP activity value of the diluted solution was found to be 218.167 U/L. Therefore, the ALP activity value of the stock solution was determined to be 218167 U/L.

(5.2) Preparation of calibrators C0 to C5

Five calibrators C1 to C5 having different protein concentrations were prepared by diluting the stock solution of the calibrator containing the polypeptide molecule 4 by the weight method based on the calibrator dilution buffer. A calibrator dilution buffer was used as the calibrator C0. The protein concentrations and ALP activity values of the six calibrators C0 to C5 are shown in Table 18.

[Table 18]

| CALIBRATOR | C0 | C1 | C2 | C3 | C4 | C5 |
|---|---|---|---|---|---|---|
| PROTEIN CONCENTRATION ($\mu$g/mL) | 0 | 0.0015771 | 0.0063085 | 0.0252340 | 0.2271062 | 0.6813187 |
| ALP ACTIVITY VALUE (U/L) | 0 | 0.5050164 | 2.0200658 | 8.0802630 | 72.722367 | 218.16710 |

(5.3) Preparation of calibration curve of protein concentration

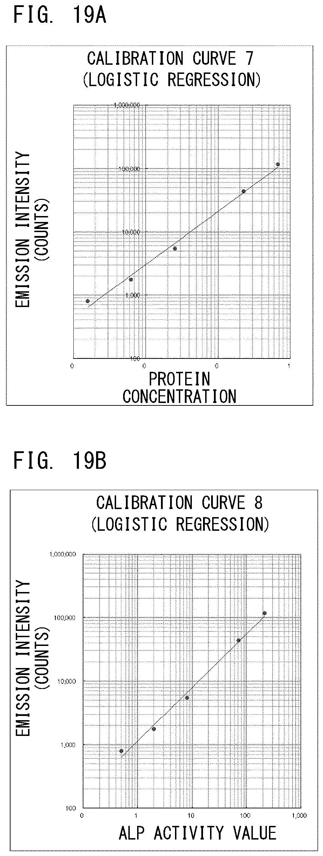

Using the above-described R1, R2, R4, and R5 reagents, the ALP activity of the calibrators C0 to C5 was measured by HI-1000, and emission intensity (count value) was acquired. The measurement was performed three times for each calibrator. The results are shown in Table 19. "AV", "SD" and "CV" in the table are the same as Table 3 in Example 1. A calibration curve was prepared on the basis of logistic regression, using the protein concentrations of the calibrators shown in Table 18 and the average values of the emission intensities of the calibrators shown in Table 19. This calibration curve is referred to as "calibration curve 7", and is shown in Fig. 19A.

[Table 19]

|  | CO | C1 | C2 | C3 | C4 | C5 |
|---|---|---|---|---|---|---|
|  | 492 | 799 | 1727 | 5472 | 41420 | 122636 |
|  | 471 | 760 | 1755 | 5412 | 45128 | 111400 |
|  | 491 | 840 | 1775 | 5516 | 44021 | 116246 |
| AV | 484.66667 | 799.66667 | 1752.3333 | 5466.6667 | 43523 | 116760.67 |
| SD | 9.6724121 | 32.663265 | 19.686431 | 42.62498 | 1554.2027 | 4601.4914 |
| CV | 2% | 4% | 1% | 1% | 4% | 4% |

The emission intensity of each calibrator shown in Table 19 was applied to a calibration curve 7 to obtain a protein concentration (μg/mL) as an actual measured value. For each calibrator, the protein concentration obtained from the emission intensity and the protein concentration (theoretical concentration) shown in Table 18 are shown in Table 20 below. As can be seen from Table 20, for the calibrators C1 to C5, the protein concentration obtained from the emission intensity using a calibration curve was found to be a value very close to the theoretical concentration. Therefore, it was shown that the polypeptide molecule 4 can be used as a standard substance for quantifying the protein concentration by immunoassay using a capture antibody, similarly to the polypeptide molecule 1.

[Table 20]

|  | CO | C1 | C2 | C3 | C4 | C5 |
|---|---|---|---|---|---|---|
| THEORETICAL CONCENTRATION (μg/mL) | 0 | 0.0015771 | 0.0063085 | 0.025234 | 0.2271062 | 0.6813187 |
|  | 3.497E-05 | 0.0015548 | 0.0062321 | 0.0254322 | 0.2191894 | 0.707057 |
|  | 0 | 0.0013601 | 0.0063741 | 0.0251221 | 0.2399584 | 0.6353008 |
|  | 3.016E-05 | 0.0017598 | 0.0064755 | 0.0256596 | 0.2337432 | 0.6660727 |
| AV | 2.171E-05 | 0.0015583 | 0.0063606 | 0.0254046 | 0.2309636 | 0.6694769 |
| SD | 1.548E-05 | 0.0001632 | 9.983E-05 | 0.0002203 | 0.0087037 | 0.0293931 |
| CV | 71% | 10% | 2% | 1% | 4% | 4% |

(5.4) Preparation of calibration curve of ALP activity value

A calibration curve was prepared based on logistic regression using the ALP activity values of the calibrators shown in Table 18 and the average values of the emission intensities of the calibrators shown in Table 19. This calibration curve is referred to as "calibration curve 8", and is shown in Fig. 19B. The emission intensity of each calibrator shown in Table 19 was applied to a calibration curve 8, and an ALP activity value (U/L) as an actual measured value was acquired. For each calibrator, the ALP activity value obtained from the emission intensity and the ALP activity value (theoretical ALP activity) shown in Table 18 are shown in Table 21 below. As can be seen from Table 21, for the calibrators C1 to C5, the ALP activity value obtained from the emission intensity using a calibration curve was a value very close to the theoretical ALP activity. Therefore, it was shown that the polypeptide molecule 4 can be used as a standard substance for quantifying ALP activity by immunoassay using a capture antibody, similarly to the polypeptide molecule 1.

[Table 21]

|  | C0 | C1 | C2 | C3 | C4 | C5 |
|---|---|---|---|---|---|---|
| THEORETICAL ALP ACTIVITY (U/L) | 0 | 0.5050164 | 2.0200658 | 8.080263 | 72.722367 | 218.1671 |
|  | 0.0111971 | 0.4978793 | 1.9955956 | 8.1437061 | 70.187284 | 226.40886 |
|  | 0 | 0.4355334 | 2.0410583 | 8.044413 | 76.837789 | 203.43159 |
|  | 0.0096565 | 0.5635076 | 2.0735388 | 8.2165343 | 74.847611 | 213.28515 |
| AV | 0.0069512 | 0.4989734 | 2.0367309 | 8.1348845 | 73.957561 | 214.3752 |
| SD | 0.0049553 | 0.052251 | 0.031967 | 0.0705446 | 2.7870467 | 9.4120465 |
| CV | 71% | 10% | 2% | 1% | 4% | 4% |

(6) Preparation of a set of calibrators containing polypeptide molecule 5

(6.1) Valuing of protein concentration and ALP activity of calibrator

The eluent obtained by affinity purification was used as a stock solution of the calibrator. The protein concentration of this stock solution was measured in the same manner as Example 1 by NanoDrop-1000. The protein concentration of the stock solution was found to be 0.624 mg/mL. The stock solution was diluted 50-fold with the calibrator dilution buffer, and the ALP activity of the diluted solution was biochemically measured in the same manner as in Example 1. The ALP activity value of the diluted solution was found to be 242.775 U/L. Therefore, the ALP activity value of the stock solution was determined to be 1213 U/L.

(6.2) Preparation of calibrators C0 to C5

Five calibrators C1 to C5 having different protein concentrations were prepared by diluting a stock solution of the calibrator containing the polypeptide molecule 5 by a weight method based on a calibrator dilution buffer. A calibrator dilution buffer was used as the calibrator C0. The protein concentrations and ALP activity values of the six calibrators C0 to C5 are shown in Table 22.

[Table 22]

| CALIBRATOR | C0 | C1 | C2 | C3 | C4 | C5 |
|---|---|---|---|---|---|---|
| PROTEIN CONCENTRATION ($\mu$g/mL) | 0 | 0.0028831 | 0.0115325 | 0.0461300 | 0.4151697 | 1.2455090 |
| ALP ACTIVITY VALUE (U/L) | 0 | 0.5619789 | 2.2479155 | 8.9916620 | 80.924958 | 242.77488 |

(6.3) Preparation of calibration curve of protein concentration

Using the above-described R1, R2, R4, and R5 reagents, the ALP activity of the calibrators C0 to C5 was measured by HI-1000, and emission intensity (count value) was acquired. The measurement was performed three times for each calibrator. The results are shown in Table 23. "AV", "SD" and "CV" in the table are the same as Table 3 in Example 1. A calibration curve was prepared based on logistic regression using the protein concentrations of the calibrators shown in Table 22 and the average values of the emission intensities of the calibrators shown in Table 23. This calibration curve is referred to as "calibration curve 9", and is shown in Fig. 20A.

[Table 23]

|  | C0 | C1 | C2 | C3 | C4 | C5 |
|---|---|---|---|---|---|---|
|  | 492 | 1244 | 3435 | 11762 | 77252 | 149358 |
|  | 471 | 1301 | 3385 | 11814 | 79319 | 156353 |
|  | 491 | 1269 | 3325 | 11795 | 80308 | 157336 |
| AV | 484.66667 | 1271.3333 | 3381.6667 | 11790.333 | 78959.667 | 154349 |
| SD | 9.6724121 | 23.328571 | 44.969125 | 21.483844 | 1273.2175 | 3551.9134 |
| CV | 2% | 2% | 1% | 0% | 2% | 2% |

The emission intensity of each calibrator shown in Table 23 was applied to a calibration curve 9 to obtain a protein concentration (μg/mL) as an actual measured value. For each calibrator, the protein concentration obtained from the emission intensity and the protein concentration (theoretical concentration) shown in Table 22 are shown in Table 24 below. As can be seen from Table 24, for the calibrators C1 to C5, the protein concentration obtained from the emission intensity using a calibration curve was found to be a value very close to the theoretical concentration. Therefore, it was shown that the polypeptide molecule 5 can be used as a standard substance for quantifying the protein concentration by immunoassay using a capture antibody, similarly to the polypeptide molecule 1.

[Table 24]

|  | C0 | C1 | C2 | C3 | C4 | C5 |
|---|---|---|---|---|---|---|
| THEORETICAL CONCENTRATION (μg/mL) | 0 | 0.0028831 | 0.0115325 | 0.04613 | 0.4151697 | 1.245509 |
|  | 1.491E-05 | 0.00257 | 0.0116088 | 0.051759 | 0.4599172 | 1.0238343 |
|  | 0 | 0.0027851 | 0.0113902 | 0.0520261 | 0.4745415 | 1.0844273 |
|  | 1.267E-05 | 0.0026641 | 0.0111285 | 0.0519285 | 0.4815717 | 1.0930274 |
| AV | 9.196E-06 | 0.0026731 | 0.0113758 | 0.0519045 | 0.4720101 | 1.0670963 |
| SD | 6.567E-06 | 8.803E-05 | 0.0001963 | 0.0001104 | 0.0090198 | 0.0307917 |
| CV | 71% | 3% | 2% | 0% | 2% | 3% |

(6.4) Preparation of calibration curve of ALP activity value

A calibration curve was prepared based on logistic regression using the ALP activity values of the calibrators shown in Table 22 and the average values of the emission intensities of the calibrators shown in Table 23. This calibration curve is referred to as "calibration curve 10", and is shown in Fig. 20B. The emission intensity of each calibrator shown in Table 23 was applied to a calibration curve 10, and an ALP activity value (U/L) as an actual measured value was acquired. For each calibrator, the ALP activity value obtained from the emission intensity and the ALP activity value (theoretical ALP activity) shown in Table 22 are shown in Table 25 below. As can be seen from Table 25, for the calibrators C1 to C5, the ALP activity value obtained from the emission intensity using a calibration curve was a value very close to the theoretical ALP activity. Therefore, it was shown that the polypeptide molecule 5 can be used as a standard substance for quantifying ALP activity by immunoassay using a capture antibody, similarly to the polypeptide molecule 1.

[Table 25]

|  | C0 | C1 | C2 | C3 | C4 | C5 |
|---|---|---|---|---|---|---|
| THEORETICAL ALP ACTIVITY (U/L) | 0 | 0.5619789 | 2.2479155 | 8.991662 | 80.924958 | 242.77488 |
|  | 0.002907 | 0.5009445 | 2.2627913 | 10.088867 | 89.647153 | 199.566 |
|  | 0 | 0.5428649 | 2.2201862 | 10.140941 | 92.497731 | 211.3768 |
|  | 0.0024706 | 0.5192892 | 2.1691708 | 10.121911 | 93.868066 | 213.05313 |
| AV | 0.0017925 | 0.5210329 | 2.2173828 | 10.11724 | 92.004316 | 207.99865 |
| SD | 0.00128 | 0.0171583 | 0.0382717 | 0.0215144 | 1.758147 | 6.0019226 |
| CV | 71% | 3% | 2% | 0% | 2% | 3% |

Example 3: Measurement using a calibrator containing polypeptide molecule 6

In Example 3, a calibrator containing the polypeptide molecule 6 was prepared from the fractions recovered and purified by SEC, and the ALP concentration was measured by ELISA. The polypeptide molecule 6 was, as shown in Fig. 9B, a fusion protein that contains, in order from the N-terminus, a signal peptide of luciferase, CD9(ECD), GS1 peptide linker, and TNAP as a series of polypeptide chains.

(1) Measurement of ALP concentration of calibrator

The ALP concentration of the polypeptide molecule 6 contained in the fraction acquired by SEC was measured by sandwich ELISA. Specific operations were as follows. A 96-well plate (Nunc, high binding ELISA plate, #436110) was washed twice with 50 mM Tris-HCl (pH7.5). The anti-CD9 antibody (Biolegend, 312102) was adjusted to a concentration of 5 μg/mL with 50 mM Tris-HCl (pH7.5) to prepare capture antibody solution. Fifty μL of the capture antibody solution was added to each well, and the content was incubated at room temperature for 2 hours while being stirred with a shaker at 400 rpm. As a control, a reagent in which control IgG was added to the R1 buffer was added to another well, and the wells were incubated in the same manner. The solution in the well was discarded and washed three times with 5 mM phosphate buffer. 200 μL of Blocking Buffer I (0.1 M TEA, 1% BSA, 0.5% Casein Na) was added to each well, and the content was incubated at room temperature for 3 hours while being stirred with a shaker at 600 rpm. As a sample, a two-fold dilution series of a fraction containing the polypeptide molecule 6 acquired by SEC was prepared. The blocking buffer I in the wells was discarded, and 100 μL of sample was added to each well. The 96-well plate was incubated at room temperature with stirring with a shaker at 600 rpm for 2.5 hours. The solution in the wells was discarded, and each well was washed three times with HISCL washing solution (300 μL/well). The ALP-labeled anti-TNAP antibody (1.06 mg/mL) was diluted 1000-fold with R1 buffer to prepare a detection antibody solution. 100 μL of the detection antibody solution was added to each well, and the content was incubated at room temperature for one hour while being stirred with a shaker at 600 rpm. The solution in the wells was discarded, and each well was washed three times with HISCL washing solution (300 μL/well). A substrate solution was prepared by mixing a HISCL R4 reagent (Sysmex Corporation) containing a substrate buffer and a HISCL R5 reagent (Sysmex Corporation) containing CDP-star at 1:2. 100 μL of the substrate solution was added to each well, and the content was incubated at room temperature for 20 minutes. Then, chemiluminescence was detected by plate reader Infinite Pro 200 (TECAN Corporation), and the ALP activity of the detection antibody bound to polypeptide molecule 6 was measured. The measurement was repeated three times, and average values of the three measured values were acquired.

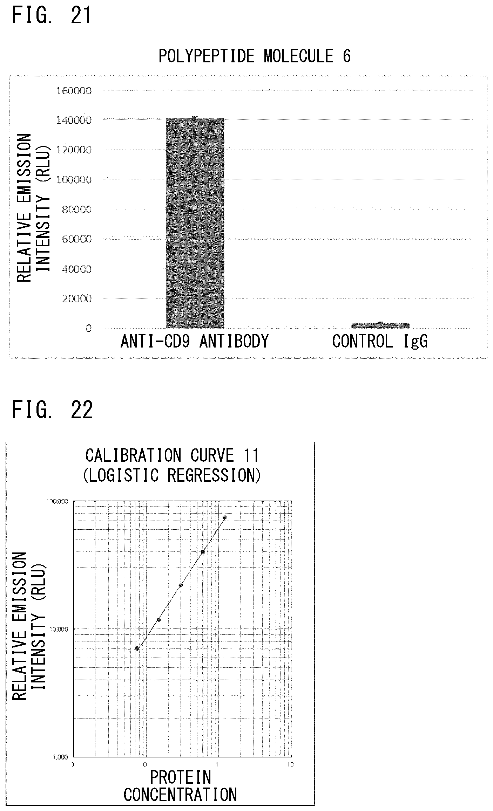

The results are shown in Fig. 21. As shown in Fig. 21, significantly high signal measured values were acquired when the calibrator was measured using anti-CD9 antibody as the capture antibody. However, when control IgG was used instead of the anti-CD9 antibody, little signal was detected. As described above, when the anti-CD9 antibody capable of specifically capturing the polypeptide molecule 6 was used, TNAP contained in the polypeptide molecule 6 could be detected. Therefore, it was shown that the polypeptide molecule 6 can be used for measurement of ALP concentration by immunoassay.

(2) Preparation of a set of calibrators

(2.1) Valuing of protein concentration of calibrator

The fraction acquired by SEC was used as a stock solution of the calibrator. The protein concentration of this stock solution was measured in the same manner as Example 1 by NanoDrop-1000. The protein concentration of the stock solution was found to be 0.115 mg/mL.

(2.2) Preparation of calibrators C0 to C5

Five calibrators C1 to C5 having different protein concentrations were prepared by diluting the stock solution of the calibrator containing the polypeptide molecule 6 by the weight method based on the calibrator dilution buffer. A calibrator dilution buffer was used as the calibrator C0. The protein concentrations of the six calibrators C0 to C5 are shown in Table 26.

[Table 26]

| CALIBRATOR | C0 | C1 | C2 | C3 | C4 | C5 |
|---|---|---|---|---|---|---|
| PROTEIN CONCENTRATION (μg/mL) | 0 | 0.075684 | 0.151367 | 0.302734 | 0.605469 | 1.210938 |

(2.3) Preparation of calibration curve of ALP concentration

In the same manner as in the aforementioned (1), the ALP concentration of the calibrators C0 to C5 was measured by sandwich ELISA method, and the relative emission intensity (RLU) was acquired. The measurement was performed three times for each calibrator. The results are shown in Table 27. "AV", "SD" and "CV" in the table are the same as Table 3 in Example 1. A calibration curve was prepared on the basis of logistic regression, using the protein concentrations of the calibrators shown in Table 26 and the average values of the relative emission intensities of the calibrators shown in Table 27. This calibration curve is called "calibration curve 11", and is shown in Fig. 22.

[Table 27]

|  | C0 | C1 | C2 | C3 | C4 | C5 |
|---|---|---|---|---|---|---|
|  | 2170 | 7519 | 12002 | 21379 | 40166 | 74931 |
|  | 2122 | 6895 | 12176 | 23346 | 40855 | 75492 |
|  | 1906 | 6636 | 11401 | 21078 | 38721 | 72944 |
| AV | 2066 | 7016.6667 | 11859.667 | 21934.333 | 39914 | 74455.667 |
| SD | 114.8216 | 370.60701 | 332.01439 | 1005.7343 | 889.23825 | 1093.1704 |
| CV | 6% | 5% | 3% | 5% | 2% | 1% |

The relative emission intensities of the calibrators shown in Table 27 were applied to a calibration curve 11 to obtain protein concentration (μg/mL) as an actual measured value. For each calibrator, the protein concentration obtained from the relative emission intensity and the protein concentration (referred to as theoretical concentration) shown in Table 26 are shown in Table 28 below. As can be seen from Table 28, for the calibrators C1 to C5, the protein concentration obtained from the relative emission intensity using a calibration curve was found to be a value very close to the theoretical concentration. This showed that the protein concentration converted from the signal measured values acquired by the immunoassay using the capture antibody and the detection antibody was almost the same as the protein concentration acquired by the absorbance measurement. Therefore, it was shown that the polypeptide molecule 6 can be used as a standard substance for quantifying the ALP concentration by immunoassay using the capture antibody and the detection antibody.

[Table 28]

|  | C0 | C1 | C2 | C3 | C4 | C5 |
|---|---|---|---|---|---|---|
| THEORETICAL CONCENTRATION (μg/mL) | 0 | 0.0756836 | 0.1513672 | 0.3027344 | 0.6054688 | 1.2109375 |
|  | 0.0015043 | 0.0830265 | 0.1529186 | 0.3018835 | 0.6097305 | 1.2100191 |
|  | 0.0008041 | 0.0733826 | 0.1556505 | 0.3335492 | 0.6212443 | 1.2200312 |
|  | 0 | 0.0693869 | 0.1434927 | 0.29705 | 0.5856338 | 1.1746412 |
| AV | 0.0007695 | 0.0752653 | 0.1506873 | 0.3108276 | 0.6055362 | 1.2015638 |
| SD | 0.0006146 | 0.0057253 | 0.0052081 | 0.0161874 | 0.0148373 | 0.019471 |
| CV | 80% | 8% | 3% | 5% | 2% | 2% |

Example 4: Measurement using a calibrator containing polypeptide molecule 7

In Example 4, a calibrator containing the polypeptide molecule 7 was prepared from the fractions recovered and purified by SEC, and the protein concentration and the ALP activity were measured. The polypeptide molecule 7 was, as shown in Fig. 9B, a fusion protein that contains, in order from the N-terminus, a signal peptide of luciferase, BIAP II, GS1 peptide linker, and an epitope region of Annexin A1 as a series of polypeptide chains.

(1) Preparation of calibrator

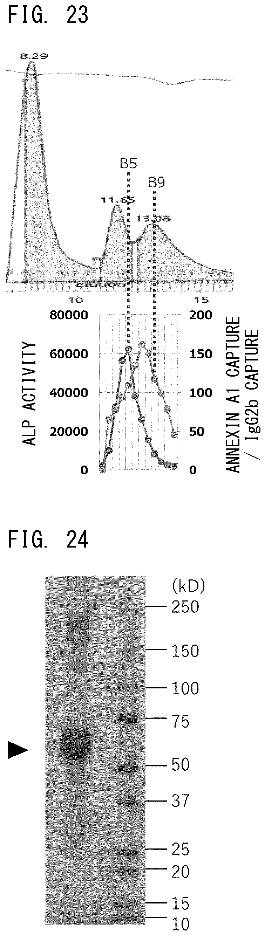

For each fraction acquired by SEC, ALP activity of each calibrator was measured by HI-1000 in the same manner as in Example 1, using the same R1, R2, R4 and R5 reagents as in Example 2. Fig. 23 illustrates results of the SEC analysis (upper chromatogram), measured results of the ALP activity of each fraction, and ratio of the measured value by the capture antibody to the measured value by the control IgG (lower graph). Referring to the graph of Fig. 23, the fraction (fraction B5) labeled with the left dashed line labeled "B5" showed the highest ALP activity. In the measurement of ALP activity, the signal measured value acquired using an R1 reagent containing a capture antibody (anti-Annexin A1 antibody) was divided by the signal measured value acquired using a reagent containing control IgG (IgG2b) instead of the R1 reagent to obtain a ratio. As a result, as shown in the graph of Fig. 23, the fractions (fractions B5 to B9) between the left dashed line and the right dashed line labeled "B9" exhibited high ratio values. Therefore, it was considered that the polypeptide molecule 7 was contained in the fractions B5 to B9. Equal amounts of these fractions were mixed, and the obtained mixture was analyzed by SDS-PAGE under reducing conditions. The result of CBB staining of the gel after electrophoresis is shown in Fig. 24. Referring to Fig. 24, it was shown that the polypeptide molecule 7 is contained in the mixture of fractions B5 to B9. A mixture of fractions B5 to B9 was used as a calibrator of Example 4 in the subsequent experiment.

(2) Preparation of a set of calibrators

(2.1) Valuing of protein concentration and ALP activity of calibrator

A mixture of the aforementioned fractions B5 to B9 was used as a stock solution of the calibrator. The protein concentration of this stock solution was measured in the same manner as Example 1 by NanoDrop-1000. The protein concentration of the stock solution was found to be 0.231 mg/mL. The stock solution was diluted 30-fold with the calibrator dilution buffer, and the ALP activity of the diluted solution was biochemically measured in the same manner as in Example 1. The ALP activity value of the diluted solution was found to be 488.865 U/L. Therefore, the ALP activity value of the stock solution was determined to be 14666 U/L.

(2.2) Preparation of calibrators C0 to C5

Five calibrators C1 to C5 having different protein concentrations were prepared by diluting the stock solution of the calibrator containing the polypeptide molecule 7 by the weight method based on the calibrator dilution buffer. A calibrator dilution buffer was used as the calibrator C0. The protein concentrations and ALP activity values of the six calibrators C0 to C5 are shown in Table 29.

[Table 29]

| CALIBRATOR | C0 | C1 | C2 | C3 | C4 | C5 |
|---|---|---|---|---|---|---|
| PROTEIN CONCENTRATION ($\mu$g/mL) | 0 | 0.0177932 | 0.0711728 | 0.2846914 | 2.562222 | 7.686667 |
| ALP ACTIVITY VALUE (U/L) | 0 | 1.1316312 | 4.5265248 | 18.106099 | 162.95489 | 488.86468 |

(2.3) Preparation of calibration curve of protein concentration

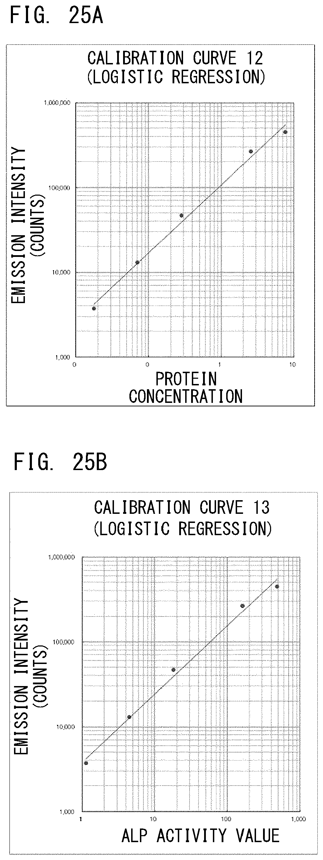

Using the above-described R1, R2, R4, and R5 reagents, the ALP activity of the calibrators C0 to C5 was measured by HI-1000, and emission intensity (count value) was acquired. The measurement was performed three times for each calibrator. The results are shown in Table 30. "AV", "SD" and "CV" in the table are the same as Table 3 in Example 1. A calibration curve was prepared based on logistic regression using the protein concentrations of the calibrators shown in Table 29 and the average values of the emission intensities of the calibrators shown in Table 30. This calibration curve is referred to as "calibration curve 12", and is shown in Fig. 25A.

[Table 30]

| | C0 | C1 | C2 | C3 | C4 | C5 |
|---|---|---|---|---|---|---|
| | 400 | 3802 | 13008 | 47028 | 266047 | 438963 |
| | 379 | 3652 | 13069 | 46812 | 260306 | 459963 |
| | 411 | 3651 | 12964 | 45832 | 270929 | 452075 |
| AV | 396.66667 | 3701.6667 | 13013.667 | 46557.333 | 265760.67 | 450333.67 |
| SD | 13.274872 | 70.947555 | 43.052939 | 520.41351 | 4341.5452 | 8661.1847 |
| CV | 3% | 2% | 0% | 1% | 2% | 2% |

The emission intensity of each calibrator shown in Table 30 was applied to calibration curve 12 to obtain the protein concentration (µg/mL) as an actual measured value. For each calibrator, the protein concentration obtained from the emission intensity and the protein concentration (theoretical concentration) shown in Table 29 are shown in Table 31 below. As can be seen from Table 31, for the calibrators C1 to C5, the protein concentration obtained from the emission intensity using a calibration curve was found to be a value very close to the theoretical concentration. Therefore, it was shown that the polypeptide molecule 7 can be used as a standard substance for quantifying the protein concentration by immunoassay using a capture antibody, similarly to the polypeptide molecule 1.

[Table 31]

|  | C0 | C1 | C2 | C3 | C4 | C5 |
|---|---|---|---|---|---|---|
| THEORETICAL CONCENTRATION (µg/mL) | 0 | 0.0177932 | 0.0711728 | 0.2846914 | 2.5622222 | 7.6866667 |
|  | 5.651E-06 | 0.0161666 | 0.0731736 | 0.3365554 | 2.9430059 | 6.1121018 |
|  | 0 | 0.01535 | 0.0735834 | 0.3347136 | 2.8563225 | 6.5819795 |
|  | 3.016E-05 | 0.0153446 | 0.0728783 | 0.32638 | 3.0175573 | 6.4029985 |
| AV | 1.194E-05 | 0.0156204 | 0.0732118 | 0.3325497 | 2.9389619 | 6.3656933 |
| SD | 1.309E-05 | 0.0003862 | 0.0002891 | 0.0044269 | 0.0658859 | 0.193632 |
| CV | 110% | 2% | 0% | 1% | 2% | 3% |

(2.4) Preparation of calibration curve of ALP activity value

A calibration curve was prepared based on logistic regression using the ALP activity values of the calibrators shown in Table 29 and the average values of the emission intensities of the calibrators shown in Table 30. This calibration curve is referred to as "calibration curve 13", and is shown in Fig. 25B. The emission intensity of each calibrator shown in Table 30 was applied to a calibration curve 13, and an ALP activity value (U/L) as an actual measured value was acquired. For each calibrator, the ALP activity value obtained from the emission intensity and the ALP activity value (theoretical ALP activity) shown in Table 29 are shown in Table 32 below. As can be seen from Table 32, for the calibrators C1 to C5, the ALP activity value obtained from the emission intensity using a calibration curve was a value very close to the theoretical ALP activity. Therefore, it was shown that the polypeptide molecule 7 can be used as a standard substance for quantifying ALP activity by immunoassay using a capture antibody, similarly to the polypeptide molecule 1.

[Table 32]

|  | C0 | C1 | C2 | C3 | C4 | C5 |
|---|---|---|---|---|---|---|
| THEORETICAL ALP ACTIVITY (U/L) | 0 | 1.1316312 | 4.5265248 | 18.106099 | 162.95489 | 488.86468 |
|  | 0.0003594 | 1.0281835 | 4.6537739 | 21.404601 | 187.17237 | 388.72385 |
|  | 0 | 0.976247 | 4.679833 | 21.287464 | 181.65939 | 418.60762 |
|  | 0.0019184 | 0.9759019 | 4.6349901 | 20.757459 | 191.91377 | 407.22461 |
| AV | 0.0007593 | 0.9934441 | 4.656199 | 21.149841 | 186.91518 | 404.85203 |
| SD | 0.0008327 | 0.0245648 | 0.0183872 | 0.2815472 | 4.1902813 | 12.314809 |
| CV | 110% | 2% | 0% | 1% | 2% | 3% |

Example 5: Measurement using a calibrator containing polypeptide molecule 8

In Example 5, a calibrator containing the polypeptide molecule 8 was prepared from the fractions recovered and purified by SEC, and the ALP concentration was measured by ELISA. As shown in Fig. 9B, the polypeptide molecule 8 was a fusion protein that contains, in order from the N-terminus, a signal peptide of luciferase, a TNAP, a GS1 peptide linker, and an epitope region of Annexin A1 as a series of polypeptide chains.

(1) Measurement of ALP concentration of calibrator

The ALP concentration of polypeptide molecule 8 contained in the fraction acquired by SEC was measured by sandwich ELISA. Specific operations were as follows. A 96-well plate (Nunc, high binding ELISA plate, #436110) was washed twice with 50 mM Tris-HCl (pH7.5). The anti-Annexin A1 antibody (Santacruz, sc-12740) was adjusted to a concentration of 8 $\mu$g/mL with 50 mM Tris-HCl (pH7.5) to prepare capture antibody solutions. Fifty $\mu$L of the capture antibody solution was added to each well, and the mixture was allowed to stand at 4° C. overnight. As a control, a reagent in which control IgG was added to 50 mM Tris-HCl (pH7.5) was added to another well, and the mixture was allowed to stand at 4° C. overnight. The solution in the well was discarded and washed three times with 5 mM phosphate buffer. 200 $\mu$L of Blocking Buffer I was added to each well, and the mixture was allowed to stand at 4° C. overnight. As a sample, a two-fold dilution series of a fraction containing the polypeptide molecule 8 acquired by SEC was prepared. The blocking buffer I in the wells was discarded, and 100 $\mu$L of sample was added to each well. The 96-well plate was allowed to stand at room temperature for 2 hours. The solution in the wells was discarded, and each well was washed three times with HISCL washing solution (300 $\mu$L/well). The ALP-labeled anti-TNAP antibody (1.06 mg/mL) was diluted 200-fold with 50 mM Tris-HCl (pH7.5) to prepare detection antibody solutions. 100 $\mu$L of the detection antibody solution was added to each well, and the mixture was allowed to stand at room temperature for 1 hour. The solution in the wells was discarded, and each well was washed three times with HISCL washing solution (300 $\mu$L/well). A substrate solution was prepared by mixing the HISCL R4 reagent (Sysmex Corporation) and the HISCL R5 reagent (Sysmex Corporation) at 1:2. 100 $\mu$L of the substrate solution was added to each well, and the mixture was allowed to stand at room temperature for 90 minutes. Then, chemiluminescence was detected by plate reader Infinite Pro 200 (TECAN Corporation), and the ALP activity of the detection antibody bound to polypeptide molecule-8 was measured. The measurement was repeated three times, and average values of the three measured values were acquired.

As a result of the measurement, a remarkably high signal measured value was acquired when the calibrator was measured using the anti-Annexin A1 antibody as the capture antibody. However, when control IgG was used instead of the anti-Annexin A1 antibody, little signal was detected. As described above, when the anti-Annexin A1 antibody capable of specifically capturing the polypeptide molecule 8 was used, TNAP contained in the polypeptide molecule 8 could be detected. Therefore, it was shown that the polypeptide molecule 8 can be used for measurement of ALP concentration by immunoassay.

(2) Preparation of a set of calibrators

(2.1) Valuing of protein concentration of calibrator

The fraction acquired by SEC was used as a stock solution of the calibrator. The protein concentration of this stock solution was measured in the same manner as Example 1 by NanoDrop-1000.

(2.2) Preparation of calibrators C0 to C5

Five calibrators C1 to C5 having different protein concentrations were prepared by diluting the stock solution of the calibrator containing the polypeptide molecule 8 by the weight method based on the calibrator dilution buffer. A calibrator dilution buffer was used as the calibrator C0. The protein concentrations and ALP activity values of the six calibrators C0 to C5 are shown in Table 33.

[Table 33]

| CALIBRATOR | C0 | C1 | C2 | C3 | C4 | C5 |
|---|---|---|---|---|---|---|
| PROTEIN CONCENTRATION ($\mu$g/mL) | 0 | 17.5 | 35 | 70 | 140 | 280 |

(2.3) Preparation of calibration curve of protein concentration

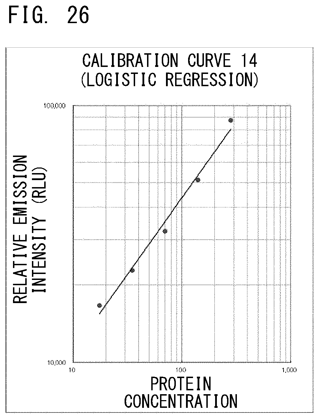

In the same manner as in the aforementioned (1), the ALP concentration of the calibrators C0 to C5 was measured by sandwich ELISA method, and the relative emission intensity (RLU) was acquired. The measurement was repeated twice for calibrator C0, and three times for calibrators C1 to C5. The results are shown in Table 34. "AV", "SD" and "CV" in the table are the same as Table 3 in Example 1. A calibration curve was prepared on the basis of logistic regression, using the protein concentrations of the calibrators shown in Table 33 and the average values of the relative emission intensities of the calibrators shown in Table 34. This calibration curve is called "calibration curve 14", and is shown in Fig. 26.

[Table 34]

|  | C0 | C1 | C2 | C3 | C4 | C5 |
|---|---|---|---|---|---|---|
|  | 14003 | 16520 | 21617 | 31891 | 50865 | 88954 |
|  | 11554 | 16436 | 21723 | 33174 | 51863 | 86093 |
|  |  | 17022 | 25049 | 32133 | 50941 | 87751 |
| AV | 12778.5 | 16659.333 | 22796.333 | 32399.333 | 51223 | 87599.333 |
| SD | 1224.5 | 258.7268 | 1593.4636 | 556.61018 | 453.6107 | 1172.9116 |
| CV | 10% | 2% | 7% | 2% | 1% | 1% |

The relative emission intensities of the calibrators shown in Table 34 were applied to a calibration curve 14 to obtain protein concentration ($\mu$g/mL) as an actual measured value. For each calibrator, the protein concentration obtained from the relative emission intensity and the protein concentration (referred to as theoretical concentration) shown in Table 33 are shown in Table 35 below. As can be seen from Table 35, for the calibrators C1 to C5, the protein concentration obtained from the relative emission intensity using a calibration curve was found to be a value very close to the theoretical concentration. This showed that the protein concentration converted from the signal measured values acquired by the immunoassay using the capture antibody and the detection antibody was almost the same as the protein concentration acquired by the absorbance measurement. Therefore, it was shown that the polypeptide molecule 8 can be used as a standard substance for quantifying the ALP concentration by immunoassay using the capture antibody and the detection antibody.

[Table 35]

|  | C0 | C1 | C2 | C3 | C4 | C5 |
|---|---|---|---|---|---|---|
| THEORETICAL CONCENTRATION ($\mu$g/mL) | 0 | 17.5 | 35 | 70 | 140 | 280 |
|  | 5.4272779 | 15.478043 | 34.755569 | 72.123327 | 139.57535 | 275.20152 |
|  | 0 | 15.151405 | 35.148597 | 76.722725 | 143.10748 | 264.90929 |
|  |  | 17.421642 | 47.379334 | 72.991622 | 139.84433 | 270.87099 |
| AV | 2.7136389 | 16.01703 | 39.0945 | 73.945891 | 140.84239 | 270.32726 |
| SD | 2.7136389 | 1.0021229 | 5.8604591 | 1.9952593 | 1.6054236 | 4.2193381 |
| CV | 100% | 6% | 15% | 3% | 1% | 2% |

[Explanation of the numerals in the Drawings]

1: Immunoassay device
10: Kit comprising a calibrator
20,30: Kit with set of calibrators
11,21,31: the first container
12,23,37: packing box
13, 24, 38: attached document
22,32: the second container
33: Third container
34: Fourth container
35: Fifth container
36: Sixth container
40: Measurement unit
41, 51: control unit
42, 53: storage unit
43, 52: communication unit

44: Measurement mechanism unit
45: Detection unit
441: Reagent holding section
442: Dispensing section
443: BF separation section
444: Reaction section
50: Analysis unit
54: Display part
55: Input unit

**Claims**

1. A method for measuring an activity of alkaline phosphatase contained in an extracellular vesicle in a sample, comprising the step of acquiring an activity value of alkaline phosphatase contained in the extracellular vesicle in the sample using a calibrator,
   wherein the calibrator comprises a polypeptide molecule comprising an amino acid sequence of alkaline phosphatase and an amino acid sequence of a membrane protein present on a surface of the extracellular vesicle.

2. The method according to claim 1, wherein the polypeptide molecule comprises an enzyme activity of alkaline phosphatase based on the polypeptide chain of the amino acid sequence of the alkaline phosphatase.

3. The method according to claim 2, further comprising the step of measuring the activity of alkaline phosphatase contained in the extracellular vesicle in the sample and the activity of the polypeptide molecule in the calibrator,
   wherein the activity value of alkaline phosphatase contained in the extracellular vesicle in the sample is acquired based on results of the measuring.

4. The method according to claim 3, further comprising the step of:

   forming on a solid phase a first complex comprising the extracellular vesicle in the sample and a capture antibody that specifically binds to the membrane protein contained in the extracellular vesicle;
   measuring a first signal generated by contacting the extracellular vesicle in the first complex with a substrate for alkaline phosphatase;
   forming on a solid phase a second complex comprising the polypeptide molecule in the calibrator and the capture antibody;
   measuring a second signal generated by contacting the polypeptide molecule in the second complex with the substrate; and
   acquiring an activity value of the alkaline phosphatase contained in the extracellular vesicle in the sample from a measured value of the first signal and a measured value of the second signal.

5. The method according to claims 1 or 2, wherein

   the alkaline phosphatase contained in the extracellular vesicle in the sample and the polypeptide molecule in the calibrator are each detected, and
   a concentration of the alkaline phosphatase contained in the extracellular vesicle in the sample is acquired based on results of the detections.

6. The method according to claim 5, wherein the polypeptide molecule is detected via a polypeptide chain represented by the amino acid sequence of the alkaline phosphatase.

7. The method according to claims 5 or 6, further comprising:

   forming on a solid phase a first complex comprising the extracellular vesicle in the sample, a capture antibody that specifically binds to a membrane protein contained in the extracellular vesicle, a detection antibody that specifically binds to alkaline phosphatase contained in the extracellular vesicle, and a labeling substance;
   measuring a first signal generated by the labeling substance contained in the first complex;
   forming on a solid phase a second complex comprising the polypeptide molecule in the calibrator, the capture antibody, the detection antibody, and the labeling substance;

measuring a second signal generated by the labeling substance contained in the second complex; and acquiring an activity value of alkaline phosphatase contained in the extracellular vesicle in the sample from measured values of the first signal and the second signal.

8. The method according to any one of claims 1 to 7, wherein the calibrator comprises a homodimer consisting of two of the polypeptide molecules.

9. The method according to any one of claims 1 to 8, wherein the amino acid sequence of the membrane protein of the polypeptide molecule is an amino acid sequence selected from the group consisting of an extracellular domain of CD9, a region from an N-terminus to a position 20 of Annexin A1, an extracellular domain of CD63, an extracellular domain of CD81, an extracellular domain of Annexin A6, and an extracellular domain of Pit 1.

10. The method according to any one of claims 1 to 9, wherein the amino acid sequence of the alkaline phosphatase contained in the polypeptide molecule is an amino acid sequence selected from the group consisting of tissue non-specific alkaline phosphatase, small intestine alkaline phosphatase, placenta alkaline phosphatase, germ cell alkaline phosphatase, and fragments thereof.

11. The method according to any one of claims 1 to 10, wherein the polypeptide molecule comprises an amino acid sequence of a peptide linker between the amino acid sequence of the alkaline phosphatase and the amino acid sequence of the membrane protein.

12. A polypeptide molecule for measuring activity or concentration of alkaline phosphatase contained in an extracellular vesicle in a sample,
the polypeptide molecule comprising a series of an amino acid sequence comprising an amino acid sequence of the alkaline phosphatase and an amino acid sequence of a membrane protein present on a surface of the extracellular vesicle.

13. A homodimer comprising the polypeptide molecule according to claim 12.

14. A calibrator for measuring activity or concentration of alkaline phosphatase contained in an extracellular vesicle in a sample,
the calibrator comprising a polypeptide molecule comprising a series of amino acid sequences comprising an amino acid sequence of alkaline phosphatase and an amino acid sequence of a membrane protein present on a surface of an extracellular vesicle.

15. A method for producing a polypeptide molecule for measuring activity or concentration of alkaline phosphatase contained in an extracellular vesicle in a sample, comprising:

culturing a cell into which an expression vector comprising a gene encoding a polypeptide molecule comprising a series of amino acid sequences comprising an amino acid sequence of alkaline phosphatase and an amino acid sequence of a membrane protein present on a surface of the extracellular vesicle is introduced; and
acquiring the polypeptide molecule expressed by the cell.

FIG. 1A

CONFIGURATION EXAMPLE 1

| MP | ALP |
|---|---|

CONFIGURATION EXAMPLE 2

| ALP | MP |
|---|---|

CONFIGURATION EXAMPLE 3

| MP1 | ALP | MP2 |
|---|---|---|

FIG. 1B

CONFIGURATION EXAMPLE 4

| MP | Linker | ALP |
|---|---|---|

CONFIGURATION EXAMPLE 5

| ALP | Linker | MP |
|---|---|---|

CONFIGURATION EXAMPLE 6

| MP1 | L1 | ALP | L2 | MP2 |
|---|---|---|---|---|

FIG. 2

FIG. 3A

FIG. 3B

FIG. 4A

FIRST SIGNAL

SUBSTRATE

EV

ALP ALP

MP

CAPTURE
ANTIBODY

SOLID PHASE

FIG. 4B

SUBSTRATE

SECOND SIGNAL

POLYPEPTIDE
MOLECULE

ALP

ALP

POLYPEPTIDE
MOLECULE

MP

MP

CAPTURE
ANTIBODY

SOLID PHASE

FIG. 5A

SUBSTRATE

FIRST SIGNAL

LABELING
SUBSTANCE
(ENZYME)

DETECTION ANTIBODY

ALP

ALP

EV

CAPTURE
ANTIBODY

MP

SOLID PHASE

FIG. 5B

SUBSTRATE

SECOND SIGNAL

LABELING SUBSTANCE
(ENZYME)

DETECTION ANTIBODY

POLYPEPTIDE
MOLECULE

ALP

ALP

POLYPEPTIDE
MOLECULE

MP

MP

CAPTURE ANTIBODY

SOLID PHASE

FIG. 6

VECTOR
EXAMPLE 1

| MP | ALP |

VECTOR
EXAMPLE 2

| ALP | MP |

VECTOR
EXAMPLE 3

| MP1 | ALP | MP2 |

VECTOR
EXAMPLE 4

| MP | Linker | ALP |

VECTOR
EXAMPLE 5

| ALP | Linker | MP |

VECTOR
EXAMPLE 6

| MP1 | L1 | ALP | L2 | MP2 |

FIG. 7

1
40    50

MEASUREMENT UNIT

CONTROL UNIT   41

STORAGE UNIT   42

COMMUNICATION UNIT   43

MEASUREMENT MECHANISM UNIT   44

REAGENT HOLDING SECTION   441

DISPENSING SECTION   442

BF SEPARATION SECTION   443

REACTION SECTION   444

DETECTION UNIT   45

ANALYSIS UNIT

CONTROL UNIT   51

COMMUNICATION UNIT   52

STORAGE UNIT   53

DISPLAY UNIT   54

INPUT UNIT   55

FIG. 8A

CONTROL UNIT 41                    CONTROL UNIT 51

START                              START

S1    MEASUREMENT VALUE
      OF SECOND SIGNAL
MEASUREMENT OF
CALIBRATOR

                                   PREPARATION OF          S2
                                   CALIBRATION CURVE

END                                END

FIG. 8B

CONTROL UNIT 41                    CONTROL UNIT 51

START                              START

S3    MEASUREMENT VALUE
      OF FIRST SIGNAL
MEASUREMENT OF SAMPLE

                                   ACQUISITION OF ALP       S4
                                   ACTIVITY VALUE OR
                                   CONCENTRATION

                                   DISPLAY ANALYSIS RESULT   S5

END                                END

# FIG. 9A

EXPRESSION
VECTOR 1

| Kozak | Luc-signal | CD9 (ECD) | Linker | BIAP II |

EXPRESSION
VECTOR 2

| Kozak | Luc-signal | CD9 (ECD) | Linker | BIAP II | Linker | Tag |

EXPRESSION
VECTOR 3

| Kozak | Luc-signal | BIAP II | Linker | CD9 (ECD) | Linker | Tag |

EXPRESSION
VECTOR 4

| Kozak | Luc-signal | BIAP II | Linker | Annexin | Linker | Tag |

EXPRESSION
VECTOR 5

| Kozak | Luc-signal | Annexin | Linker | BIAP II | Linker | Annexin | Linker | Tag |

EXPRESSION
VECTOR 6

| Kozak | Luc-signal | CD9 (ECD) | Linker | TNAP |

EXPRESSION
VECTOR 7

| Kozak | Luc-signal | BIAP II | Linker | Annexin |

EXPRESSION
VECTOR 8

| Kozak | Luc-signal | TNAP | Linker | Annexin |

# FIG. 9B

POLYPEPTIDE
MOLECULE 1

| Luc-signal | CD9 (ECD) | Linker | BIAP II |

POLYPEPTIDE
MOLECULE 2

| Luc-signal | CD9 (ECD) | Linker | BIAP II | Linker | Tag |

POLYPEPTIDE
MOLECULE 3

| Luc-signal | BIAP II | Linker | CD9 (ECD) | Linker | Tag |

POLYPEPTIDE
MOLECULE 4

| Luc-signal | BIAP II | Linker | Annexin | Linker | Tag |

POLYPEPTIDE
MOLECULE 5

| Luc-signal | Annexin | Linker | BIAP II | Linker | Annexin | Linker | Tag |

POLYPEPTIDE
MOLECULE 6

| Luc-signal | CD9 (ECD) | Linker | TNAP |

POLYPEPTIDE
MOLECULE 7

| Luc-signal | BIAP II | Linker | Annexin |

POLYPEPTIDE
MOLECULE 8

| Luc-signal | TNAP | Linker | Annexin |

FIG. 10

FIG. 11

EP 4 621 065 A1

FIG. 12

POLYPEPTIDE MOLECULE 1

FIG. 13A

50

FIG. 13B

CALIBRATION CURVE 2
(LOGISTIC REGRESSION)

FIG. 14A

## FIG. 14B

## FIG. 15

POLYPEPTIDE MOLECULE

## FIG. 16A

POLYPEPTIDE MOLECULE 2

FIG. 16B

POLYPEPTIDE MOLECULE 3

FIG. 16C

POLYPEPTIDE MOLECULE 4

FIG. 16D

POLYPEPTIDE MOLECULE 5

FIG. 17A

CALIBRATION CURVE 3
(LOGISTIC REGRESSION)

FIG. 17B

CALIBRATION CURVE 4
(LOGISTIC REGRESSION)

FIG. 18A

CALIBRATION CURVE 5
(LOGISTIC REGRESSION)

FIG. 18B

CALIBRATION CURVE 6
(LOGISTIC REGRESSION)

FIG. 19A

CALIBRATION CURVE 7
(LOGISTIC REGRESSION)

FIG. 19B

CALIBRATION CURVE 8
(LOGISTIC REGRESSION)

FIG. 20A

CALIBRATION CURVE 9
(LOGISTIC REGRESSION)

PROTEIN
CONCENTRATION

FIG. 20B

CALIBRATION CURVE 10
(LOGISTIC REGRESSION)

ALP ACTIVITY VALUE

FIG. 21

POLYPEPTIDE MOLECULE 6

FIG. 22

# FIG. 23

# FIG. 24

FIG. 25A

CALIBRATION CURVE 12
(LOGISTIC REGRESSION)

FIG. 25B

CALIBRATION CURVE 13
(LOGISTIC REGRESSION)

FIG. 26

CALIBRATION CURVE 14
(LOGISTIC REGRESSION)

Europäisches
Patentamt

European
Patent Office

Office européen
des brevets

**EUROPEAN SEARCH REPORT**

**Application Number**

EP 25 16 4075

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | EP 4 343 327 A1 (SYSMEX CORP [JP]) 27 March 2024 (2024-03-27) <br><br> * the whole document * <br> * in particular: * <br> * examples 1,2 * <br> ----- | 1-6, 8-10, 12-14 | INV. C12Q1/42 |
| X | MOURA SILIO LIMA ET AL: "The activity of alkaline phosphatase in breast cancer exosomes simplifies the biosensing design", BIOSENSORS AND BIOELECTRONICS, ELSEVIER SCIENCE LTD, UK, AMSTERDAM , NL, vol. 198, 23 November 2021 (2021-11-23), XP086909166, ISSN: 0956-5663, DOI: 10.1016/J.BIOS.2021.113826 [retrieved on 2021-11-23] * the whole document * * in particular: * * figure 1 * * section 2.5; page 2 * * section 2.6; page 3 * ----- | 7 | |
| A | WENDY FITZGERALD ET AL: "Extracellular vesicles generated by placental tissues ex vivo: A transport system for immune mediators and growth factors", AMERICAN JOURNAL OF REPRODUCTIVE IMMUNOLOGY, vol. 80, no. 1, 4 May 2018 (2018-05-04), page e12860, XP055547900, US ISSN: 1046-7408, DOI: 10.1111/aji.12860 * the whole document * ----- | 7 | **TECHNICAL FIELDS SEARCHED (IPC)** <br><br> C12Q <br> G01N |

-/--

The present search report has been drawn up for all claims

| Place of search | Date of completion of the search | Examiner |
|---|---|---|
| The Hague | 1 August 2025 | Tuynman, Antonin |

Europäisches
Patentamt

European
Patent Office

Office européen
des brevets

# EUROPEAN SEARCH REPORT

**Application Number**

EP 25 16 4075

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHEN XINZHU ET AL: "Development of a single-chain variable fragment-alkaline phosphatase fusion protein-based direct competitive enzyme-linked immunosorbent assay for furaltadone metabolite in ctenopharyngodon idellus", FOOD AND AGRICULTURAL IMMUNOLOGY., vol. 32, no. 1, 1 January 2021 (2021-01-01), pages 403-418, XP093301421, GB ISSN: 0954-0105, DOI: 10.1080/09540105.2021.1957781 * abstract * | 11,15 | |
| X | US 2021/394183 A1 (TANG SINDY K Y [US] ET AL) 23 December 2021 (2021-12-23) | 12-14 | |
| Y | * paragraph [0108] * | 11,15 | |
| | | | TECHNICAL FIELDS SEARCHED (IPC) |

The present search report has been drawn up for all claims

| Place of search | Date of completion of the search | Examiner |
|---|---|---|
| The Hague | 1 August 2025 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                              
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 16 4075

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2025

| Patent document cited in search report | | Publication date | Patent family member(s) | | Publication date |
|---|---|---|---|---|---|
| EP 4343327 | A1 | 27-03-2024 | CN | 117741144 A | 22-03-2024 |
| | | | EP | 4343327 A1 | 27-03-2024 |
| | | | JP | 2024046503 A | 03-04-2024 |
| | | | US | 2024118272 A1 | 11-04-2024 |
| US 2021394183 | A1 | 23-12-2021 | US | 2021394183 A1 | 23-12-2021 |
| | | | WO | 2020102429 A1 | 22-05-2020 |

EPO FORM P0459

For more details about this annex : see Official Journal of the European Patent Office, No. 12/82

**REFERENCES CITED IN THE DESCRIPTION**

**Patent documents cited in the description**

- US 8993247 B
- US 6406899 B

- JP H1254868 B

**Non-patent literature cited in the description**

- **MANES T. et al.** *J.Biol.Chem.*, 1998, vol. 273, 23353-23360